# EUROPEAN PATENT APPLICATION

(11) **EP 4 545 919 A1**
(43) Date of publication of application: **30.04.2025**
(21) Application number: 23842177.0
(22) Date of filing: 12.07.2023
(51) Int. Cl.: G01J 1/44, A61B 5/024

(54) **LIGHT SOURCE DIMMING METHOD AND ELECTRICAL DEVICE**

(30) Priority: 22.07.2022 CN 202210868093
(71) Applicant: Huawei Technologies Co., Ltd., Shenzhen, Guangdong 518129 (CN)
(72) Inventor: YANG, Sulin, Shenzhen, Guangdong 518129 (CN); WANG, Dan, Shenzhen, Guangdong 518129 (CN); YANG, Ruhui, Shenzhen, Guangdong 518129 (CN); LIU, Zongwen, Shenzhen, Guangdong 518129 (CN); HUANG, Jingli, Shenzhen, Guangdong 518129 (CN); LIN, Yue, Shenzhen, Guangdong 518129 (CN); AI, Wansen, Shenzhen, Guangdong 518129 (CN); LI, Dong, Shenzhen, Guangdong 518129 (CN)
(74) Representative: Pfenning, Meinig & Partner mbB
(86) International application number: PCT/CN2023/106953
(87) International publication number: WO 2024/017112

(57) **Abstract**

This application discloses a light source dimming method and an electronic device. The electronic device includes a light emitter, an optical-to-electrical converter, a trans-impedance amplifier, a dimming module, and a DC adjustment module. The dimming module is configured to: determine a first photocurrent of the optical-to-electrical converter based on an obtained digital signal and a preset gain of the trans-impedance amplifier; determine a current transmission ratio between the light emitter and the optical-to-electrical converter based on the first photocurrent and a first drive current of the light emitter; determine, based on the current transmission ratio, a second drive current of the light emitter, and a second photocurrent of the optical-to-electrical converter when the light emitter is driven by using the second drive current to emit light; and when it is determined that the second photocurrent is within an optimal working range of a target current, adjust a DC current of the DC adjustment module based on the second photocurrent, for a difference between the second photocurrent and the DC current to be within an optimal working range of the trans-impedance amplifier in a high-gain mode. In an entire dimming process, dimming duration is greatly shortened, and therefore, quick dimming is implemented.

## Description

This application claims priority to Chinese Patent Application No. 202210868093.4, filed with the China National Intellectual Property Administration on July 22, 2022 and entitled "LIGHT SOURCE DIMMING METHOD AND ELECTRONIC DEVICE", which is incorporated herein by reference in its entirety.

### TECHNICAL FIELD

Embodiments of the present invention relate to the field of electronic devices, and in particular, to a light source dimming method and electronic device.

### BACKGROUND

Photoplethysmography (photoplethysmography, PPG) is used to obtain a plethysmogram in an optical manner. A common implementation is as follows: Light emitted by a light-emitting diode (light-emitting diode, LED) is radiated to skin, and then an optical signal is transmitted to an optical-to-electrical converter (photo-diode, PD) through transmission or reflection. The PD converts the received optical signal into an electrical signal, and sends the electrical signal to an analog front end (analog front end, AFE) chip. The AFE chip converts the electrical signal into a PPG digital signal through a first analog-to-digital converter (analog-to-digital converter, ADC) for output.

Generally, a PPG sensor is used in a wearable sports device (for example, a sports watch). In a motion scenario, relative positions between skin and the PPG sensor change. When the LED emits light with preset light intensity, strength of an optical signal received by the PD may change greatly. When the optical signal received by the PD is excessively strong or excessively weak, performance of the PPG sensor deteriorates. A target current working range is generally preset for the PD, so that the PPG sensor works within a range of best performance. When a PD current exceeds the target current working range, the PD current may return to the target current working range by adjusting a drive current of the LED. This adjustment process is referred to as a dimming process.

In an existing light source dimming method, a micro controller unit (micro controller unit, MCU) is generally used. The MCU reads, through an AFE interface (generally an SPI or I2C), a PPG digital signal output by an ADC, and determines, based on the PPG digital signal, whether a receive channel of an AFE chip works within an optimal range. If the receive channel of the AFE chip deviates from the optimal range, the MCU controls, through the foregoing interface, an LED driver of the AFE chip to adjust a drive current of an LED, so that the receive channel of the AFE chip works in the optimal range. However, a dimming process is long and needs to take a long time to become stable. Especially, in a rapid fluctuation scenario, the PPG sensor cannot quickly track the PD current. Consequently, the receive channel of the AFE chip is saturated or performance of the receive channel of the AFE chip is sharply reduced.

### SUMMARY

Embodiments of this application provide a light source dimming method and an electronic device, to implement quick dimming.

According to a first aspect, an embodiment of this application provides an electronic device. The electronic device may include a first light emitter, a first optical-to-electrical converter, a first trans-impedance amplifier, a first analog-to-digital converter, a dimming module, and a first DC adjustment module, where the other end of the first analog-to-digital converter is electrically connected to one end of the dimming module, the other end of the dimming module is electrically connected to the first light emitter and one end of the first DC adjustment module, the other end of the first DC adjustment module is electrically connected to one end of the first optical-to-electrical converter, the other end of the first optical-to-electrical converter is electrically connected to one end of the first trans-impedance amplifier, and the other end of the first trans-impedance amplifier is electrically connected to one end of the first analog-to-digital converter.

The dimming module is configured to: determine a first photocurrent of the first optical-to-electrical converter based on a digital signal output by the first analog-to-digital converter and a preset gain of the first trans-impedance amplifier (optionally, the preset gain includes a preset current value of the IDAC 1); determine a current transmission ratio between the first light emitter and the first optical-to-electrical converter based on the first photocurrent and a first drive current of the first light emitter; determine, based on the current transmission ratio, a second drive current of the first light emitter, and a second photocurrent of the first optical-to-electrical converter when the first light emitter is driven by using the second drive current to emit light; and when it is determined that the second photocurrent is within an optimal working range of a target current, adjust a DC current of the first DC adjustment module based on the second photocurrent, for a difference between the second photocurrent and the DC current to be within an optimal working range of the first trans-impedance amplifier in a high-gain mode.

Alternatively, a second drive current of the first light emitter is determined based on the current transmission ratio, and a second photocurrent of the first optical-to-electrical converter and a DC current of the first DC adjustment module are determined when the first light emitter is driven by using the second drive current to emit light, so that a difference between the second photocurrent and the DC current is within an optimal working range of the first trans-impedance amplifier in a high-gain mode.

It can be learned that the electronic device provided in this embodiment of this application may complete dimming of the light emitter in a slot of each cycle, between slots, or in a sub-slot of each slot. In this way, in an entire dimming process, dimming duration is greatly shortened, and quick dimming is implemented. Especially, in a scenario of fast fluctuation (for example, violent movement), a photocurrent of the optical-to-electrical converter can be quickly tracked to ensure performance of the electronic device.

In a specific implementation, the electronic device further includes a drive chip. The drive chip includes a first driver, the first trans-impedance amplifier, the first analog-to-digital converter, the dimming module, the first DC adjustment module, and a first current digital-to-analog converter. The other end of the dimming module is electrically connected to one end of the first driver, the other end of the first driver is electrically connected to the first light emitter, the other end of the first DC adjustment module is electrically connected to one end of the first current digital-to-analog converter, another end of the first current digital-to-analog converter is electrically connected to one end of the first optical-to-electrical converter, and the other end of the first optical-to-electrical converter is electrically connected to still another end of the first current digital-to-analog converter.

Alternatively, (a single-end connection method is used between a PD and a TIA. To be specific, an anode of the PD is grounded, and a cathode of the PD is connected to an input of the trans-impedance amplifier, as shown in the figure in the comments) the other end of the IDAC is electrically connected to one end of the PD and one end of the TIA.

In a specific implementation, the dimming module is configured to: determine, based on the current transmission ratio and a target photocurrent, the second drive current that is of the first light emitter and that is needed when the target photocurrent is reached; or determine, based on the current transmission ratio and a pre-stored correspondence between a drive current and a power, the second drive current of the first light emitter.

In a specific implementation, the dimming module is further configured to: when a difference between the second photocurrent and the target photocurrent is greater than a first threshold, control the first DC adjustment module to adjust the DC current, and obtain an adjustment value of the DC current of the first DC adjustment module, where there is a correspondence among the adjustment value, the second photocurrent, the target photocurrent, and a convergence threshold of the optimal working range of the target photocurrent, and the first threshold is a DC adjustment trigger threshold.

The adjustment value may be a DC current value of the first DC adjustment module after adjustment, or may be a difference between DC current values of the first DC adjustment module before and after adjustment.

The first threshold may be understood as a threshold for triggering DC adjustment (adjustment of an IDAC current). If a difference between a PD current and a DC current exceeds the threshold, the DC current is adjusted.

In a specific implementation, the dimming module is further configured to: when a difference between the second photocurrent and the DC current of the first DC adjustment module is greater than a second threshold, control the first DC adjustment module to adjust the DC current, and obtain an adjustment value of the DC current of the first DC adjustment module, where there is a correspondence among the adjustment value, the second photocurrent, the DC current of the first DC adjustment module, and a convergence threshold of the optimal working range of the target photocurrent.

The adjustment value may be a DC current value of the first DC adjustment module after adjustment, or may be a difference between DC current values of the first DC adjustment module before and after adjustment.

The second threshold may be understood as a threshold for triggering DC adjustment (adjustment of an IDAC current). If a difference between a PD current and a DC current exceeds the threshold, the DC current is adjusted.

In a specific implementation, the dimming module is further configured to: determine, in an m^{th} slot of an X^{th} cycle, that the second photocurrent exceeds the optimal working range of the target current, where both X and m are positive integers greater than or equal to 1; or determine, in an m^{th} slot of each cycle, that the second photocurrent exceeds the optimal working range of the target photocurrent; or after the current transmission ratio is measured in a 1^{st} slot, determine, in another slot based on a third drive current generated in the 1^{st} slot, that a photocurrent corresponding to the third drive current exceeds the optimal working range of the target photocurrent; or after the current transmission ratio is measured in a 1^{st} sub-slot of each slot, determine, based on the current transmission ratio and in a slot of a first cycle to which the 1^{st} sub-slot belongs or a slot of a second cycle to which the 1^{st} sub-slot belongs, that the second photocurrent exceeds the optimal working range of the target current; or when working is performed in one or more slots of each cycle work a dimming trigger threshold, determine the current transmission ratio, and determine that a current transmission ratio and a second photocurrent that are in another slot exceed the optimal working range of the target current.

In a specific implementation, the dimming module is further configured to: determine, in an n^{th} slot of the X^{th} cycle, that the difference between the second photocurrent and the DC current exceeds an optimal working range of a TIA, where both X and n are positive integers greater than or equal to 1; or determine, in an n^{th} slot of each cycle, that the difference between the second photocurrent and the DC current exceeds an optimal working range of a TIA.

In some specific implementations, the electronic device further includes an i^{th} optical-to-electrical converter, an i^{th} trans-impedance amplifier, an i^{th} analog-to-digital converter, an i^{th} DC adjustment module, and an i^{th} current digital-to-analog converter, where i is a positive integer greater than or equal to 2; and each optical-to-electrical converter is electrically connected to any trans-impedance amplifier and current digital-to-analog converter, and each trans-impedance amplifier is electrically connected to any analog-to-digital converter.

In some specific implementations, the electronic device further includes a j^{th} driver and a k^{th} light emitter, and both j and k are positive integers greater than or equal to 2; and each driver is electrically connected to p light emitters, where p is a positive integer greater than or equal to 1.

In a specific implementation, in a specific slot, one or more drivers drive one or more light emitters.

In a specific implementation, the electronic device may further include a memory, where the memory is electrically connected to at least one analog-to-digital converter and is configured to: receive and store a digital signal output by the at least one analog-to-digital converter.

According to a second aspect, a light source dimming method is provided, and is applied to an electronic device. The electronic device includes: one or more processors; one or more memories; one or more light emitters; one or more optical-to-electrical converters; and one or more DC adjustment modules. The method includes: in response to a first current adjustment signal, controlling the light emitter to output a first optical signal, and detecting a second optical signal obtained when the first optical signal is transmitted or reflected by a to-be-detected object; determining, based on the second optical signal, a first photocurrent corresponding to the second optical signal; obtaining a current transmission ratio between the light emitter and the optical-to-electrical converter based on the first photocurrent and a first drive current of the light emitter; determining, based on the current transmission ratio, a second drive current of the light emitter and a second photocurrent of the optical-to-electrical converter when the light emitter is driven by using the second drive current to emit light; and when it is determined that the second photocurrent is within an optimal working range of a target current, adjusting a DC current of the DC adjustment module based on the second photocurrent, for a difference between the second photocurrent and the DC current to be within an optimal working range of the first trans-impedance amplifier in a high-gain mode.

The dimming module is configured to: determine a first photocurrent of the first optical-to-electrical converter based on a digital signal output by the first analog-to-digital converter and a preset gain of the first trans-impedance amplifier (optionally, the preset gain includes a preset current value of the IDAC 1); determine a current transmission ratio between the first light emitter and the first optical-to-electrical converter based on the first photocurrent and a first drive current of the first light emitter; determine, based on the current transmission ratio, a second drive current of the first light emitter, and a second photocurrent of the first optical-to-electrical converter when the first light emitter is driven by using the second drive current to emit light; and when it is determined that the second photocurrent is within an optimal working range of a target current, adjust a DC current of the first DC adjustment module based on the second photocurrent, for a difference between the second photocurrent and the DC current to be within an optimal working range of the first trans-impedance amplifier in a high-gain mode.

Alternatively, a second drive current of the first light emitter is determined based on the current transmission ratio, and a second photocurrent of the first optical-to-electrical converter and a DC current of the first DC adjustment module are determined when the first light emitter is driven by using the second drive current to emit light, so that a difference between the second photocurrent and the DC current is within an optimal working range of the first trans-impedance amplifier in a high-gain mode.

It can be learned that the electronic device provided in this embodiment of this application may complete dimming of the light emitter in a slot of each cycle, between slots, or in a sub-slot of each slot. In this way, in an entire dimming process, dimming duration is greatly shortened, and quick dimming is implemented. Especially, in a scenario of fast fluctuation (for example, violent movement), a photocurrent of the optical-to-electrical converter can be quickly tracked to ensure performance of the electronic device.

In a specific implementation, the electronic device is further configured to: determine, based on the current transmission ratio and a target photocurrent, the second drive current that is of the light emitter and that is needed when the target photocurrent is reached; or determine, based on the current transmission ratio and a pre-stored correspondence between a drive current and a power, the second drive current of the light emitter.

In some embodiments, after the electronic device determines, based on the current transmission ratio, the second drive current of the light emitter, and the second photocurrent of the optical-to-electrical converter when the light emitter is driven by using the second drive current to emit light, the method may further include: The electronic device obtains a second current transmission ratio (CTR 2 for short) between an LED and the PD 1 based on the second photocurrent and the second drive current of the LED.

In this way, steps S1104 and S1105 may be repeatedly performed to continue to obtain, based on the second current transmission ratio between the LED and the PD 1 and the PD target current, a third drive current that is of the LED and that is needed when the PD target current is reached, and a third PD current of the PD 1 when the LED is driven by using the third drive current to emit light, until an N^{th} (N=1, 2, ...) PD current is within an optimal working range of the PD target current.

In some embodiments, after the electronic device obtains the second current transmission ratio (CTR 2 for short) between the LED and the PD 1 based on the second photocurrent and the second drive current of the LED, the method may further include: The electronic device determines whether a second PD current (for example, the second photocurrent) exceeds the optimal working range of the PD target current (for example, the target photocurrent). If the second PD current does not exceed the optimal working range of the PD target current, the electronic device determines, based on the CTR 1 and the PD target current, the second drive current of the LED and the second PD current of the PD when the LED is driven by using the second drive current to emit light; or if the second PD current exceeds the optimal working range of the PD target current, the electronic device drives the LED to emit light by using the second drive current.

Optionally, when the second PD current is within the optimal working range of the PD target current, the electronic device obtains a second current transmission ratio (CTR 2 for short) between the LED and a PD based on the second photocurrent and the second drive current of the LED, and assigns the CTR 2 to the CTR 1 (if the CTR 2 is not calculated above), or assigns the CTR 2 to the CTR 1 (if the CTR 2 is calculated above).

In some embodiments, the method further includes: The electronic device controls a DC adjustment module of a PPG sensor to set an initial DC current. In an n^{th} PRF cycle (PRF_n for short), the electronic device controls the DC adjustment module of the PPG sensor to drive an IDAC by using the specified initial DC current. The electronic device obtains a PD current received in the n^{th} PRF cycle. In an (n+1)^{th} PRF cycle (PRF_n+1 for short), the electronic device controls the DC adjustment module of the PPG sensor to drive the IDAC by using a PD current received in the (n+1)^{th} PRF cycle as a DC current in this cycle.

For example, a plurality of PRF cycles are used as a basis of a calculation operation. For example, it is assumed that 10 PRF cycles are used as a basis, the IDAC is set to work at a first DC current value in the 1^{st} to 10^{th} PRF cycles. PD current values in the 1^{st} to 10^{th} PRF cycles are calculated to obtain an average value. In 11^{th} to 20^{th} PRF cycles, the average value of the PD current values in the 1^{st} to 10^{th} PRF cycles is used as a DC current value for working. In this way, the DC current can be adjusted more stably.

In some embodiments, the method further includes: The electronic device controls the DC adjustment module of the PPG sensor to set a first DC current, so that the IDAC outputs the first DC current. The first DC current may be represented as I_{DC_O}. The electronic device determines whether a difference between the first DC current and a PD current is less than or equal to a first threshold. An expression of the first threshold may be: |I_{PD}_₂-I_{DC_O}|≤ε. If the difference between the first DC current and the PD current is less than or equal to the first threshold, the electronic device controls the DC adjustment module of the PPG sensor to set the first DC current. If the difference between the first DC current and the PD current is greater than the first threshold, the electronic device controls the DC adjustment module of the PPG sensor to adjust the DC current.

There may be two implementations in which the electronic device determines the difference between the first DC current and the PD current. Details are as follows.

Implementation 1: The electronic device may determine the difference in any slot of a current PRF cycle (a same PRF cycle) in a current PRF cycle, and then set any DC current value in the current PRF cycle (the same PRF cycle), that is, determine the difference in a prediction manner, and set the DC current value in advance.

Implementation 2: Within or after one PRF cycle, the electronic device calculates, in any slot of a current PRF cycle, the difference between the PD current and the first DC current, and determines a DC current value in any slot of a next PRF cycle.

In some embodiments, the method further includes: The electronic device controls the DC adjustment module of the PPG sensor to set a first DC current, so that the IDAC outputs the first DC current. The first DC current may be represented as I_{DC_O}. The electronic device determines whether a difference between the first DC current and a PD current is less than or equal to a first threshold. An expression of the first threshold may be: |I_{PD_2}-I_{DC_O}|≤ε. If the difference between the first DC current and the PD current is less than or equal to the first threshold, the electronic device controls the DC adjustment module of the PPG sensor to set the first DC current, so that the IDAC outputs the first DC current. If the difference between the first DC current and the PD current is greater than the first threshold, the electronic device controls the DC adjustment module of the PPG sensor to adjust the DC current, so that the IDAC outputs a second DC current, where the second DC current is a value obtained through calculation based on previous N PD currents, for example, an average value or a tendency predicted value.

In a specific implementation, the electronic device is further configured to: when a difference between the second photocurrent and the target photocurrent is greater than a first threshold, control the DC adjustment module to adjust the DC current, and obtain an adjustment value of the DC current of the first DC adjustment module, where there is a correspondence among the adjustment value, the second photocurrent, the target photocurrent, and a convergence threshold of the optimal working range of the target photocurrent, and the first threshold is a DC adjustment trigger threshold.

In a specific implementation, the electronic device is further configured to: when a difference between the second photocurrent and the DC current of the DC adjustment module is greater than a second threshold, control the DC adjustment module to adjust the DC current, and obtain an adjustment value of the DC current of the DC adjustment module, where there is a correspondence among the adjustment value, the second photocurrent, the DC current of the DC adjustment module, and a convergence threshold of the optimal working range of the target photo current.

In a specific implementation, the electronic device is further configured to: determine, in an m^{th} slot of an X^{th} cycle, that the second photocurrent exceeds the optimal working range of the target current, where both X and m are positive integers greater than or equal to 1; or determine, in an m^{th} slot of each cycle, that the second photocurrent exceeds the optimal working range of the target photocurrent; or after the current transmission ratio is measured in a 1^{st} slot, determine, in another slot based on a third drive current generated in the 1^{st} slot, that a photocurrent corresponding to the third drive current exceeds the optimal working range of the target photocurrent; or after the current transmission ratio is measured in a 1^{st} sub-slot of each slot, determine, based on the current transmission ratio and in a slot of a first cycle to which the 1^{st} sub-slot belongs or a slot of a second cycle to which the 1^{st} sub-slot belongs, that the second photocurrent exceeds the optimal working range of the target current; or when working is performed in one or more slots of each cycle within a dimming trigger threshold, determine the current transmission ratio, and determine that a current transmission ratio and a second photocurrent that are in another slot exceed the optimal working range of the target current.

In a specific implementation, the electronic device is further configured to: determine, in an n^{th} slot of the X^{th} cycle, that the difference between the second photocurrent and the DC current exceeds an optimal working range of a TIA, where both X and n are positive integers greater than or equal to 1; or determine, in an n^{th} slot of each cycle, that the difference between the second photocurrent and the DC current exceeds an optimal working range of a TIA.

According to a third aspect, a light source dimming method is provided, and is applied to an electronic device. The electronic device may include: one or more processors, one or more memories, one or more light emitters, one or more optical-to-electrical converters, and one or more DC adjustment modules. The method may include: In response to a first current adjustment signal, the electronic device controls the light emitter to output a first optical signal, and detect a second optical signal obtained when the first optical signal is transmitted or reflected by a to-be-detected object. The electronic device determines, based on the second optical signal, a first photocurrent corresponding to the second optical signal. The electronic device obtains a first current transmission ratio (CTR 1 for short) between the LED and the PD 1 based on the first photocurrent and a first drive current of the LED. The electronic device determines, based on the CTR 1, a second drive current of the LED and a second PD current of a PD when the LED is driven by using the second drive current to emit light. The electronic device obtains a second current transmission ratio (CTR 2 for short) between the LED and the PD 1 based on the second photocurrent and the second drive current of the LED. The electronic device determines whether the second PD current (for example, the second photocurrent) exceeds an optimal working range of a PD target current (for example, the target photocurrent). If the second PD current does not exceed the optimal working range of the PD target current, the electronic device controls a DC adjustment module of a PPG sensor to set a DC current based on the second PD current, so that an IDAC outputs the specified DC current. If the second PD current exceeds the optimal working range of the PD target current, the electronic device determines, based on the CTR 1, a second drive current of the LED and a second PD current of the PD when the LED is driven by using the second drive current to emit light. The electronic device determines whether a difference between a DC current and a PD current is less than or equal to a first threshold. An expression of the first threshold may be: |I_{PD_2}-I_{DC_O}|≤ε. If the difference between the first DC current and the PD current is less than or equal to the first threshold, the electronic device controls the DC adjustment module of the PPG sensor to set the DC current based on the second PD current, so that the IDAC outputs the specified DC current. If the difference between the first DC current and the PD current is greater than the first threshold, the electronic device controls the DC adjustment module of the PPG sensor to adjust the DC current.

There may be two implementations in which the electronic device determines the difference between the first DC current and the PD current. Details are as follows.

Implementation 1: The electronic device may determine the difference in any slot of a current PRF cycle (a same PRF cycle) in a current PRF cycle, and then set any DC current value in the current PRF cycle (the same PRF cycle), that is, determine the difference in a prediction manner, and set the DC current value in advance.

Implementation 2: Within or after one PRF cycle, the electronic device calculates, in any slot of a current PRF cycle, the difference between the PD current and the first DC current, and determines a DC current value in any slot of a next PRF cycle.

In a specific implementation, step S1609 is specifically: determining in an n^{th} slot of an X^{th} cycle, whether a difference between the second PD current and a DC current of a DC Cancel is within an optimal working range of a TIA, where both X and n are positive integers greater than or equal to 1; or determining in an n^{th} slot of each cycle, whether a difference between the second PD current and a DC current of a DC Cancel is within an optimal working range of a TIA.

Optionally, when the second PD current is within the optimal working range of the PD target current, the electronic device obtains a second current transmission ratio (CTR 2 for short) between the LED and a PD based on the second photocurrent and the second drive current of the LED, and assigns the CTR 2 to the CTR 1 (if the CTR 2 is not calculated above), or assigns the CTR 2 to the CTR 1 (if the CTR 2 is calculated above).

According to a fourth aspect, a computer-readable storage medium is provided, including computer instructions. When the computer instructions are run on a terminal, the terminal is enabled to perform the method according to the foregoing aspects and any one of the possible implementations of the foregoing aspects.

According to a fifth aspect, a computer program product is provided. When the computer program product runs on a computer, the computer is enabled to perform the method according to the foregoing aspects and any one of the possible implementations of the foregoing aspects.

According to a sixth aspect, a chip system is provided, including a processor. When the processor executes instructions, the processor performs the method according to the foregoing aspects and any one of the possible implementations of the foregoing aspects.

For specific implementations and corresponding technical effects of the embodiments in the second aspect to the sixth aspect, refer to specific implementations and technical effects of the first aspect.

### BRIEF DESCRIPTION OF DRAWINGS

To describe technical solutions in embodiments of this application more clearly, the following briefly introduces the accompanying drawings for describing embodiments or the conventional technology. It is clear that the accompanying drawings in the following descriptions show merely some embodiments of this application, and a person of ordinary skill in the art may still derive other drawings from these accompanying drawings without creative efforts.
FIG. 1 is a diagram of a structure of an electronic device according to an embodiment of this application;
FIG. 2 is a diagram of a structure of a PPG sensor;
FIG. 3 is a diagram of a transmission optical path of light emitted by an LED in a transmissive PPG sensor according to an embodiment of this application;
FIG. 4 is a diagram of a reflection optical path of light emitted by an LED in a reflective PPG sensor according to an embodiment of this application;
FIG. 5 is a diagram of a structure of another PPG sensor;
FIG. 6 is a diagram of a working time sequence of the PPG sensor shown in FIG. 5;
FIG. 7A is a diagram of a structure of a PPG sensor according to an embodiment of this application;
FIG. 7B is a diagram of a structure of a PPG sensor according to an embodiment of this application;
FIG. 8 is a diagram of a process of LED dimming and DC adjustment according to an embodiment of this application;
FIG. 9 is a diagram of a correspondence between output optical power of a light source and a drive current of the light source according to an embodiment of this application;
FIG. 10 is a diagram of a structure of a PPG sensor according to an embodiment of this application;
FIG. 11 is a schematic flowchart of a light source dimming method according to an embodiment of this application;
FIG. 12 is a schematic flowchart of a light source dimming method according to an embodiment of this application;
FIG. 13 is a schematic flowchart of a light source dimming method according to an embodiment of this application;
FIG. 14 is a schematic flowchart of a light source dimming method according to an embodiment of this application;
FIG. 15 is a schematic flowchart of a light source dimming method according to an embodiment of this application; and
FIG. 16A and FIG. 16B are a schematic flowchart of a light source dimming method according to an embodiment of this application.

### DESCRIPTION OF EMBODIMENTS

In descriptions of embodiments of this application, unless otherwise specified, "/" means "or". For example, A/B may represent A or B. In this specification, "and/or" describes only an association relationship for describing associated objects and represents that three relationships may exist. For example, A and/or B may represent the following three cases: Only A exists, both A and B exist, and only B exists.

The following terms "first" and "second" are merely intended for a purpose of description, and shall not be understood as an indication or implication of relative importance or implicit indication of the number of indicated technical features. Therefore, a feature limited by "first" or "second" may explicitly or implicitly include one or more features. In the description of the embodiment of this application, unless otherwise stated, "a plurality of" means two or more than two.

In addition, in embodiments of this application, the word "example" or "for example" is used to represent giving an example, an illustration, or a description. Any embodiment or design solution described as "example" or "for example" in embodiments of this application should not be interpreted as being more preferred or advantageous than another embodiment or design solution. Exactly, use of the word "example", "for example", or the like is intended to present a related concept in a specific manner.

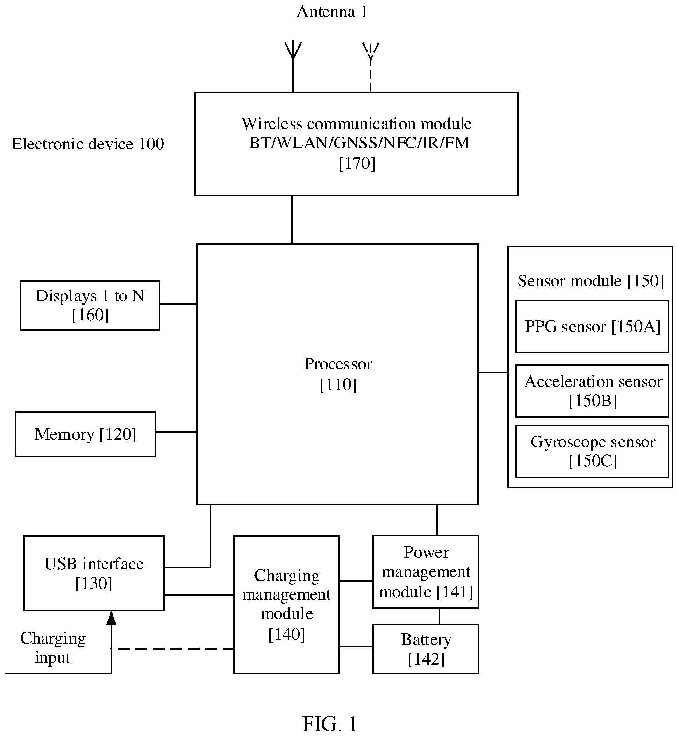

FIG. 1 is a diagram of a structure of an electronic device 100.

The electronic device 100 may include a processor 110, a memory 120, a universal serial bus (universal serial bus, USB) interface 130, a charging management module 140, a power management module 141, a battery 142, a sensor module 150, a display 160, an antenna 1, a wireless communication module 170, and the like. The sensor module 150 may include a PPG sensor 150A, an acceleration sensor 150B, a gyroscope sensor 150C, and the like.

It may be understood that, the structure illustrated in embodiments of the present invention constitutes no specific limitation on the electronic device 100. In some other embodiments of this application, the electronic device 100 may include more or fewer components than those shown in the figure, or some components may be combined, or some components may be split, or there may be a different component layout. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent devices, or may be integrated into one or more processors.

The controller may generate an operation control signal based on instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data that has been used or cyclically used by the processor 110. If the processor 110 needs to use the instructions or the data again, the processor 110 may directly invoke the instructions or the data from the memory. This avoids repeated access and reduces waiting time of the processor 110, thereby improving system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

It may be understood that an interface connection relationship between the modules shown in embodiments of the present invention is merely an example for description, and does not constitute a limitation on the structure of the electronic device 100. In some other embodiments of this application, alternatively, the electronic device 100 may use interface connection manners different from those in the foregoing embodiment or use a combination of a plurality of interface connection manners.

The memory 120 may be configured to store computer-executable program code. The executable program code includes instructions. The memory 120 may include a program storage area and a data storage area. The program storage area may store an operating system, an application required by at least one function (for example, a voice playing function or an image playing function), and the like. The data storage area may store data (for example, audio data or a phone book) created in a process of using the electronic device 100, and the like. In addition, the memory 120 may include a high-speed random access memory, or may include a nonvolatile memory, for example, at least one disk storage device, a flash memory, or a universal flash storage (universal flash storage, UFS). The processor 110 runs instructions stored in the memory 120 and/or instructions stored in the memory disposed in the processor, to perform various function applications of the electronic device 100 and data processing.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive wireless charging input through a wireless charging coil of the electronic device 100. The charging management module 140 may further supply power to the electronic device through the power management module 141 while charging the battery 142.

The power management module 141 is configured to connect to the battery 142, the charging management module 140, and the processor 110. The power management module 141 receives input of the battery 142 and/or the charging management module 140, to supply power to the processor 110, the internal memory 121, the display 160, the wireless communication module 170, and the like. The power management module 141 may be further configured to monitor parameters such as a battery capacity, a battery cycle count, and a battery health status (an electric leakage or impedance). In some other embodiments, the power management module 141 may alternatively be disposed in the processor 110. In some other embodiments, the power management module 141 and the charging management module 140 may alternatively be disposed in a same component.

The PPG sensor 150A may obtain an optical signal transmitted or reflected by a to-be-detected object. In some embodiments, the PPG sensor 150A may be in contact with human tissue, receive an optical signal transmitted or reflected by the human tissue, and generate a PPG digital signal based on the received optical signal. The processor 110 may implement a function of adjusting a drive current of the LED based on the PPG digital signal generated by the PPG sensor 150A.

The acceleration sensor 150B may detect a value of an acceleration of the electronic device 100 in each direction (usually on three axes). When the electronic device 100 is still, a magnitude and a direction of gravity may be detected. The acceleration sensor 150B may be further configured to identify a posture of the electronic device, and is applied to an application such as switching between a landscape mode and a portrait mode or a pedometer.

The gyroscope sensor 150C may be configured to determine a moving posture of the electronic device 100. In some embodiments, angular velocities of the electronic device 100 around three axes (namely, axes x, y, and z) may be determined via the gyroscope sensor 150C. The gyroscope sensor 150C may be configured to implement image stabilization during photographing. For example, when a shutter is pressed, the gyroscope sensor 150C detects an angle at which the electronic device 100 jitters, calculates, based on the angle, a distance for which a lens module needs to compensate, and allows a lens to cancel the jitter of the electronic device 100 through reverse motion, to implement image stabilization. The gyroscope sensor 150C may also be used in a navigation scenario and a somatic game scenario.

A wireless communication function of the electronic device 100 may be implemented through the antenna 1, the wireless communication module 170, the modem processor, the baseband processor, and the like.

The antenna 1 is configured to: transmit and receive electromagnetic wave signals. Each antenna in the electronic device 100 may be configured to cover one or more communication frequency bands. Different antennas may be further multiplexed, to improve antenna utilization. In some other embodiments, the antenna may be used in combination with a tuning switch.

The electronic device 100 may implement a display function through the GPU, the display 160, the application processor, and the like. The GPU is a microprocessor for image processing, and is connected to the display 160 and the application processor. The GPU is configured to: perform mathematical and geometric computation, and render an image. The processor 110 may include one or more GPUs that execute program instructions to generate or change display information.

The display 160 is configured to display an image, a video, and the like. The display 160 includes a display panel. The display panel may be a liquid crystal display (liquid crystal display, LCD), an organic light-emitting diode (organic light-emitting diode, OLED), an active-matrix organic light emitting (active-matrix organic light emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 160, where N is a positive integer greater than 1.

The wireless communication module 170 may provide a solution, applied to the electronic device 100, to wireless communication including a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 170 may be one or more devices integrating at least one communication processor module. The wireless communication module 170 receives an electromagnetic wave through the antenna 1, performs frequency modulation and filtering on an electromagnetic wave signal, and sends a processed signal to the processor 110. The wireless communication module 170 may further receive a to-be-sent signal from the processor 110, perform frequency modulation and amplification on the signal, and convert the signal into an electromagnetic wave for radiation through the antenna 1.

In some embodiments, the antenna 1 is coupled to the wireless communication module 170 of the electronic device 100, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

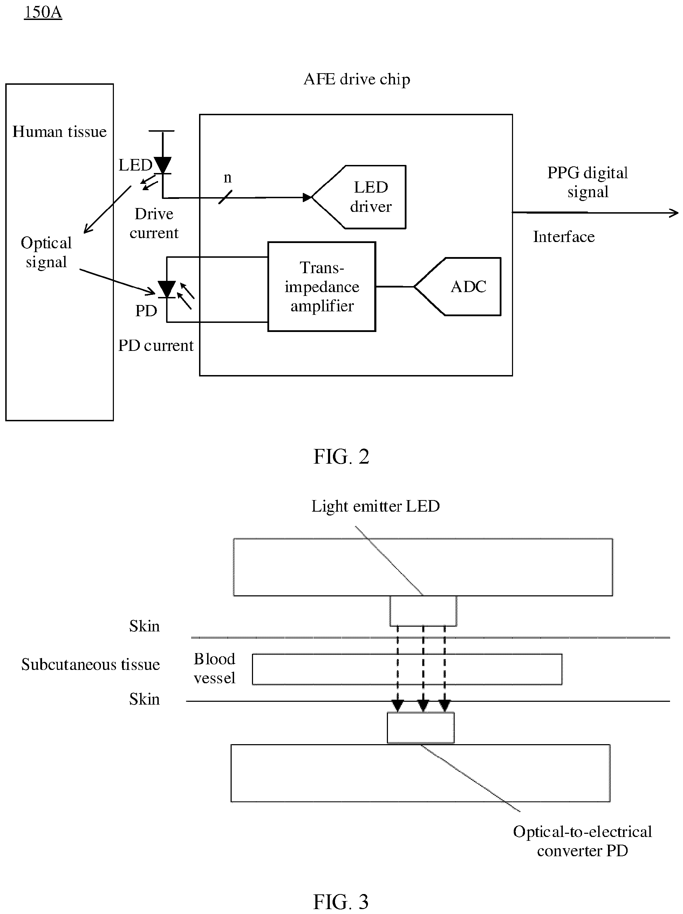

FIG. 2 is a diagram of a structure of a PPG sensor according to an embodiment of this application. As shown in FIG. 2, the PPG sensor 150A may include an AFE, a light emitter (for example, an LED), and an optical-to-electrical converter PD. The AFE includes an LED driver (configured to: set an LED drive current and drive the LED to emit light), a trans-impedance amplifier (trans-impedance amplifier, where the TIA is configured to: connect to a PD and perform current-to-voltage conversion and amplification on a photocurrent received by the PD), and an ADC (analog-to-digital converter), where the LED driver and the trans-impedance amplifier are integrated into a same chip. Alternatively, the LED driver and the trans-impedance amplifier may be disposed in two different chips. The AFE is configured to set a drive current for the LED to drive the LED to emit light. The AFE is further configured to convert, via the ADC, an electrical signal converted from an optical signal received by the PD into a PPG digital signal for output. After an optical signal emitted by the LED is irradiated to the skin, the optical signal is transmitted to the PD through scattering or reflection. The PD converts the received optical signal into an electrical signal and sends the electrical signal to the AFE. The TIA of the AFE amplifies the electrical signal and converts the amplified electrical signal into a PPG digital signal via the ADC for output. The electrical signal may include a photocurrent, namely, a PD current. An AFE chip in the following refers to an AFE or AFE function.

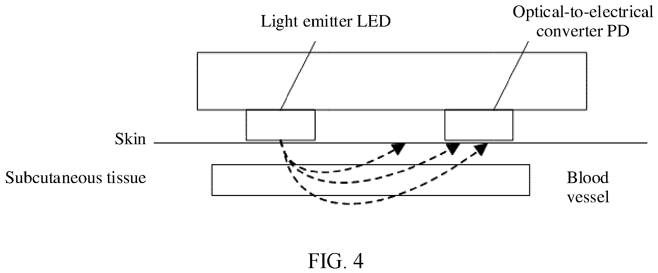

There may be two PPG sensor types: a transmissive PPG sensor and a reflective PPG sensor. FIG. 3 is a diagram of a transmission optical path of an optical signal emitted by an LED in a transmissive PPG sensor according to an embodiment of this application. As shown in FIG. 3, a feature of the transmissive PPG sensor is that an LED and a PD are located on two sides of a to-be-detected object. For example, the LED of the transmissive PPG sensor is disposed on one side of the to-be-detected object (for example, human tissue), and the PD of the transmissive PPG sensor is disposed on the other side of the to-be-detected object, so that light emitted by the LED is transmitted through the human tissue (for example, a finger). The PD receives an optical signal transmitted through the human tissue, and converts the optical signal into an electrical signal. For example, the transmissive PPG sensor is used in a fingertip oximeter commonly used in a hospital. FIG. 4 is a diagram of a reflection optical path of an optical signal emitted by an LED in a reflective PPG sensor according to an embodiment of this application. As shown in FIG. 4, a feature of the reflective PPG sensor is that an LED and a PD are located on a side of a to-be-detected object. For example, the LED and the PD of the transmissive PPG sensor are disposed on a same side of the to-be-detected object (for example, human tissue), so that light emitted by the LED enters the human tissue, and then is received by the PD through reflection by the human tissue, and the PD converts the optical signal into an electrical signal. For example, the reflective PPG sensor is used in a wearable detection product, for example, a watch or a wristband detection product.

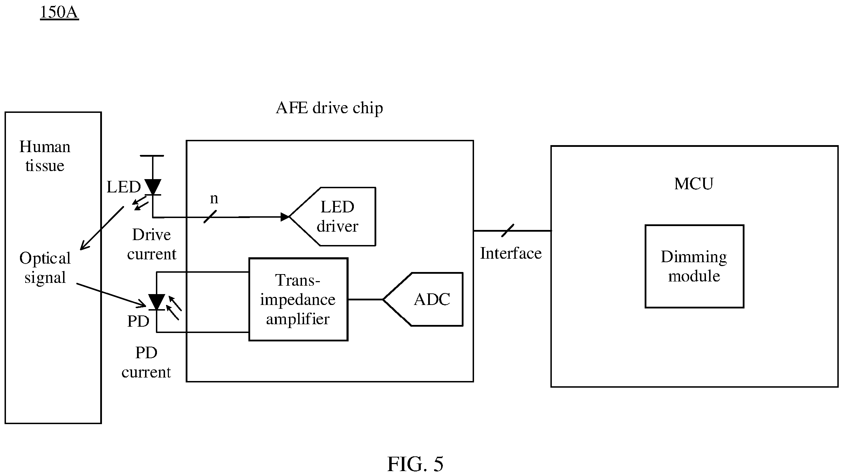

FIG. 5 is a diagram of a structure of a PPG sensor according to an embodiment of this application. As shown in FIG. 5, the PPG sensor 150A may further include an MCU. A PPG digital signal converted by an AFE chip via an ADC can be output to the MCU through an interface. The MCU reads the PPG digital signal output by the ADC through the interface (generally an SPI or I2C) of the AFE chip, and determines whether a receive channel of the AFE chip works within an optimal range based on the PPG digital signal. If the receive channel deviates from the optimal range, the MCU controls, through the foregoing interface, an LED driver of the AFE chip to adjust an LED drive current, so that the receive channel of the AFE chip works in the optimal range.

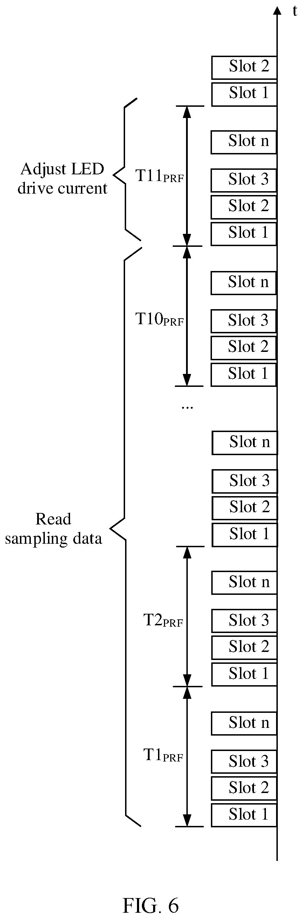

Specifically, FIG. 6 is a diagram of a working time sequence of a PPG sensor according to an embodiment of this application. As shown in FIG. 6, a working cycle of the PPG sensor is usually T_{PRF}. A detection frequency corresponding to the cycle T_{PRF} is a pulse repetition frequency (pulse repetition frequency, PRF). One T_{PRF} usually includes one or more slots (slots). In one slot, the AFE chip drives the LED to emit light, and one or more PDs receive an optical signal emitted by the LED. In addition, the MCU in an electronic device (for example, a wearable device) needs to process data collected by another sensor, and the wearable device has a constraint of low power consumption. Therefore, usually, the MCU reads a PPG digital signal (or referred to as sampling data) of the AFE chip once every n (for example, a 1^{st} to a 10^{th}) T_{PRFs}. The MCU performs calculation and determining based on the read sampling data, to determine whether an LED drive current needs to be adjusted. If the LED drive current needs to be adjusted, the MCU controls the LED driver in the AFE chip to adjust the LED drive current. In this way, the AFE chip adjusts the LED drive current in a next T_{PRF} (namely, an 11^{th} T_{PRF}) cycle, and then tracks whether an adjusted PD current works in the optimal range. If the adjusted PD current does not work in the optimal range, the AFE chip continues to adjust the LED drive current until the PD current works in the optimal range of the receive channel of the AFE chip. However, a dimming process is long and needs to take a long time to become stable. Especially, in a rapid fluctuation (for example, violent motion) scenario, the PPG sensor cannot quickly track the PD current. Consequently, the receive channel of the AFE chip is saturated or performance of the receive channel of the AFE chip is sharply reduced.

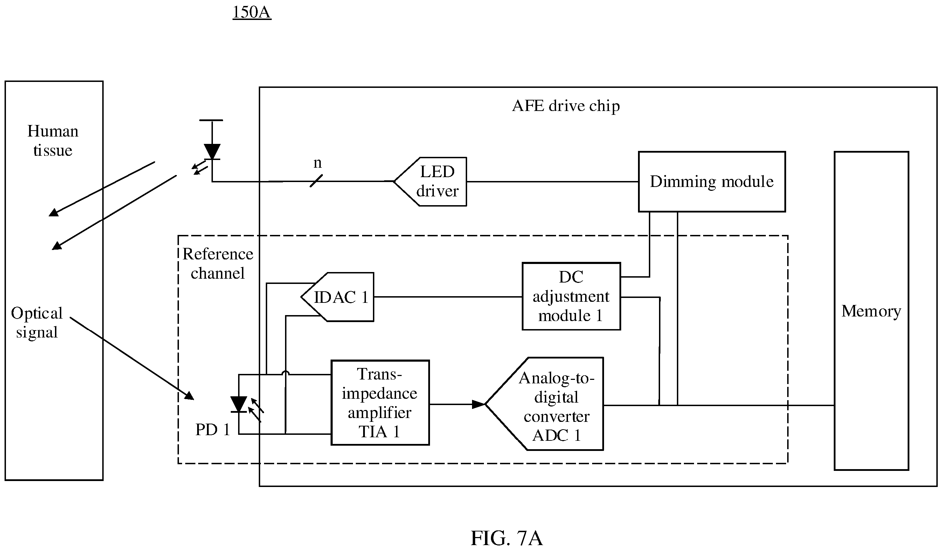

FIG. 7A is a diagram of a structure of an electronic device according to an embodiment of this application. As shown in FIG. 7A, the electronic device may include the PPG sensor 150A, to implement quick dimming and shorten dimming duration. In addition to the light emitter (for example, the LED), the PD (for example, a PD 1 shown in FIG. 7A), the TIA (for example, a TIA 1 shown in FIG. 7A), the ADC (for example, an ADC 1 shown in FIG. 7A), and the LED driver, the PPG sensor 150A further includes a dimming module, a DC adjustment module 1, and a current digital-to-analog converter (current digital-to-analog converter or current mode digital-to-analog converter, IDAC) module 1. The other end of the ADC 1 is electrically connected to one end of the dimming module. The other end of the dimming module is electrically connected to one end of the LED driver, or may be electrically connected to one end of the DC adjustment module 1. The other end of the DC adjustment module 1 is electrically connected to one end of the IDAC 1. The other end of the IDAC 1 module is electrically connected to the PD 1. The dimming module is configured to control the LED driver based on a PPG digital signal output by the ADC 1, to adjust an LED drive current, so that a PD current of the PD 1 is within a range of a PD target current. In addition, the PD and the TIA may be electrically connected directly or electrically connected through a switch circuit (for example, a multi-channel selection circuit MUX), and the PD and the IDAC 1 may be electrically connected directly or electrically connected through a switch circuit (for example, a multi-channel selection circuit MUX).

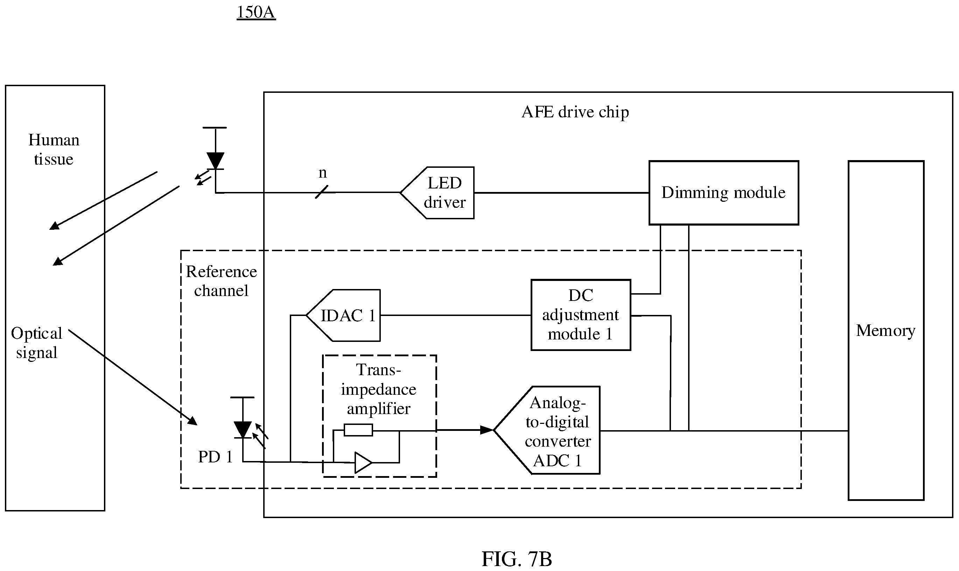

There may be two output ends of the IDAC in FIG. 7A. Because currents are output in a differential form, one output end is a source (current source), and the other output end is a current sink (current sink). In addition, FIG. 7B is a diagram of a structure of an electronic device according to an embodiment of this application. As shown in FIG. 7B, the PD is connected to the TIA in a single-end connection manner. To be specific, an anode of the PD is grounded, a cathode of the PD is connected to an input of a trans-impedance amplifier, and the other end of the IDAC is electrically connected to one end of the PD and one end of the TIA. An output end of the IDAC may be a sink or a source.

Specifically, the dimming module obtains a first PPG digital signal from the ADC 1. The dimming module calculates a first PD current (for example, the first photocurrent) of the PD 1 based on the first PPG digital signal and a preset gain of the TIA 1. The dimming module may obtain a first current transmission ratio (CTR 1 for short) between the LED and the PD 1 based on a first drive current of the LED and the first PD current. The dimming module may further obtain, based on the first current transmission ratio between the LED and the PD 1 and a PD target current (for example, the target photocurrent), a second drive current that is of the LED and that is needed when the PD target current is reached, and a second PD current (for example, the second photocurrent) of the PD 1 when the LED is driven by using the second drive current to emit light.

After the dimming module obtains the second PD current, because there is an error, for example, an error of optical-to-electrical conversion efficiency of the LED, or an error like a noise on a receive circuit, the dimming module needs to further determine whether the second PD current exceeds an optimal working range of the PD target current. If the second PD current is within the optimal working range of the PD target current, the DC adjustment module may adjust a DC current of the DC adjustment module 1 based on the second PD current, so that the AFE chip of the PPG sensor works within an optimal range at a high gain (that is, a difference between the PD current and the DC current is within the optimal working range of the AFE chip in a TIA high-gain mode). If the second PD current exceeds the optimal working range of the PD target current, the dimming module calculates a current transmission ratio between the LED and the PD 1 based on the second PD current and the second drive current, and continues to calculate, based on the second current transmission ratio between the LED and the PD 1 and the PD target current, a third drive current that is of the LED and that is needed when the PD target current is reached, and a third PD current of the PD 1 when the LED is driven by using the third drive current to emit light, until an N^{th} (N=1, 2, ...) PD current is within the optimal working range of the PD target current.

A specific implementation of DC adjustment in the PPG sensor 150A provided in this embodiment of this application is described below.

Implementation 1: When a difference between the second PD current and the PD target current is greater than a first threshold (the first threshold is a DC adjustment trigger threshold), the dimming module controls the DC adjustment module 1 to adjust the DC current, and obtains an adjustment value of the DC current of the DC adjustment module 1. There is a correspondence among the adjustment value, the second PD current, the PD target current, and a convergence threshold of the optimal working range of the AFE chip. For example, an expression of the correspondence may be |I_{PD_2}-(I_{PD_O}+ΔI_{dC})|≤ε, and c≤b. I_{PD_2} is the second PD current; I_{PD_O} is the PD target current; ΔI_{dc} is the adjustment value of I_{dc}; an initial value of I_{dc} may be set to I_{PD_O}; ε is an optimal working range or an optimal working threshold of the receive circuit; and b is the DC adjustment trigger threshold.

Implementation 2: When a difference between the second PD current and the DC current of the DC adjustment module is greater than a second threshold, the dimming module controls the DC adjustment module to adjust the DC current, and obtains an adjustment value of the DC current of the DC adjustment module. There is a correspondence among the adjustment value, the second PD current, the DC current of the DC adjustment module, and a convergence threshold of the optimal working range of the AFE chip. For example, an expression of the correspondence may be |I_{PD_2}-(I_{DC_O}+ΔI_{dc})|≤ε. I_{PD_2} is the second PD current; I_{DC_O} is a DC current value before adjustment; ΔI_{dc}=I_{PD_2}-I_{DC_O}; a DC current value after adjustment is I_{DC}=(I_{DC_O}+ΔI_{dc}); and ε is an optimal working range or an optimal working threshold of the receive circuit.

The adjustment value may be a DC current value of the first DC adjustment module after adjustment, or may be a difference between DC current values of the first DC adjustment module before and after adjustment.

Each of the first threshold and the second threshold may be understood as a threshold for triggering DC adjustment (adjusting an IDAC current). If a difference between a PD current and a DC current exceeds the threshold, the DC current is adjusted.

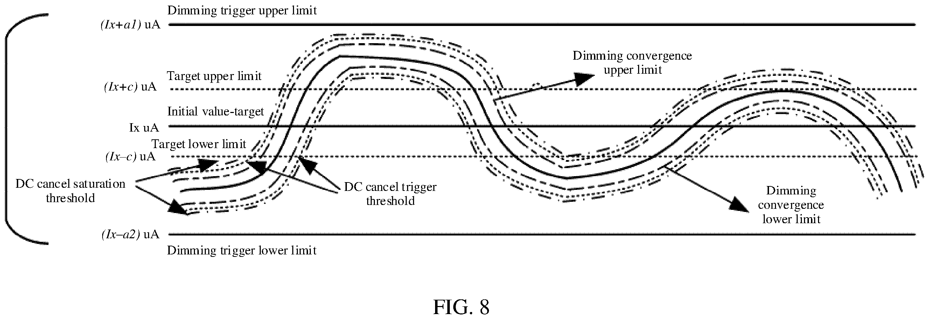

In addition, it should be noted that there are trigger thresholds in a dimming procedure and a DC_offset adjustment procedure. Specifically, one is a dimming trigger threshold (lx+/-a in FIG. 8), and the other is a DC adjustment trigger threshold DC_o+/-b (a DC cancel trigger threshold is marked in the figure). There are two convergence thresholds. One is an I_{pd} target current convergence threshold (corresponding to an initial target threshold [lx-c, lx+c] or dimming convergence upper and lower limits in FIG. 8), and the other is an optimal working range or optimal working threshold of the receive circuit (the receive circuit including the TIA and the ADC). The optimal working range or the optimal working threshold of the receive circuit is for |I_{PD_2}-(I_{DC_O}tΔI_{dc})|. The Ipd target current convergence threshold may be preset. For example, [I_{pd_o}-x, I_{pd_o}+x], I_{pd_o}, and x are preset values. If I_{PD}_₂-(I_{DC_O}+ΔI_{dc})] is set to ε, ε may be preferably less than or equal to x. If a photocurrent Ipd received by the PD exceeds dimming trigger upper and lower limits, dimming is enabled (an LED current is adjusted). If a photocurrent Ipd received by the PD is within dimming trigger upper and lower limits, namely [lx-a2, 1x+a1], only DC adjustment may be performed, and LED photocurrent adjustment is not performed.

In another case, for example, the first threshold is a dimming trigger threshold (a threshold for triggering adjustment of an LED drive current), and if the first threshold is exceeded, LED drive current adjustment is enabled, so that the PD target current returns to the optimal working range. The second threshold is a DC adjustment threshold (a threshold for triggering adjustment of the DC current), and if the second threshold is exceeded, DC adjustment is enabled. The first threshold is customized based on a difference between the photocurrent received by the PD and the PD target current (for example, the first threshold is +/-5 uA), or is customized based on the PD target current (for example, the first threshold is I_{pd_o}+/-5 uA, where I_{pd_o} is the PD target current).

The DC current is adjusted for a receive channel. After a subtraction operation is performed between the DC current and the photocurrent received by the PD, a net input current input to one end of the TIA is within a specified range (the optimal range of the receive circuit), which is usually [-i uA, i uA], and i is related to a gain of the TIA. For example, when the TIA uses a 1.8 V power supply, the gain is set to 100K ohms (100,000 ohms), and i may be set to 4. In this case, the optimal working range of the receive current including the TIA and the ADC, that is, an optimal working range of the net input current of the TIA is [-4 uA, 4 uA]. The DC adjustment module (for example, including an offset cancel function or a DC cancel control function) monitors, in time, whether a current input to the TIA (a current difference between the PD current and the IDAC current) falls within the range of [-4 uA, 4 uA]. If the current exceeds the range, an input of the IDAC is adjusted, so that the current input to the TIA falls within the range of [-4 uA, 4 uA].

In conclusion, a diagram of an adjustment process shown in FIG. 8 may be obtained by using the foregoing LED dimming and DC adjustment methods. It is assumed that a is a dimming trigger threshold, b is a DC_offset adjustment trigger threshold, and c is a dimming convergence threshold. lx-a is a dimming trigger lower limit, namely, (lx-a2) uA shown in FIG. 8; lx+a is a dimming trigger upper limit, namely, (lx+a1) uA shown in FIG. 8, where a1 and a2 may be the same or may be different; lx is an initial target current or an initial value of a target current or a target current in a dimming process; lx is equivalent to I_{PD_O}; and I_{DC_O}-b is a DC_offset adjustment trigger lower limit, and I_{DC_O}+b is a DC_offset adjustment trigger upper limit. I_{DC_O}-c is a dimming convergence lower limit, and I_{DC_O}+c is a dimming convergence upper limit. In this case, after dimming and DC_offset adjustment are performed, a PD current value falls within a range of [I_{DC}__{O}-c, I_{DC_O}+c]. In addition, a gain of the TIA 1 and the DC adjustment trigger threshold are set, so that the TIA and the ADC of the receive channel of the AFE chip can work in the optimal working range. Alternatively, after dimming (LED current adjustment) is performed, a PD current value falls within a range of [I_{DC_o}-c, I_{DC_O}+c] or [I_{PD_O}-c, I_{PD_O}+c]. As the PPG sensor runs, the PD current value falls within [lx-a2, lx-a1]. Within [lx-a2, lx-a1], the TIA (with a set gain) and the ADC of the receive channel of the AFE chip work in the optimal working range through DC_offset adjustment. Alternatively, after dimming (LED current adjustment) is performed, a PD current value falls within a range of [I_{DC_o}-c, I_{DC_O}+c]. As the PPG sensor runs, the PD current value exceeds the range of [lx-a2, lx-a1]. If it is detected that the PD current value exceeds the range of [lx-a2, lx-a1], the dimming module adjusts the LED drive current, so that the PD current value returns to the range of [I_{DC_o-}c, I_{DC_O}+c], or the dimming module adjusts the LED drive current, so that the PD current returns to the range of [I_{PD_O}-c, I_{PD_O}+c]. Herein, I_{DC_O} refers to a target specified value of a DC_offset current or a specified value of an Idac output current. I_{DC_O} may be fixed or change with a change of a PD photocurrent received in one or more historical PRF cycles. For example, I_{DC_O} in an (N+1)^{th} PRF cycle may be an I_{PD} value of a PD photocurrent in an N^{th} PRF cycle or an average value or a tendency prediction value of PD photocurrents in (N-j)^{th}, ..., and (N-1)^{th} (j=1, 2, 3, ...) PRF cycles. The LED dimming and DC current adjustment may have the following cases.
1. For LED dimming, it may be determined, based on a normal working slot or a dedicated slot, whether dimming is required in the following. Details are as follows.

In a specific implementation, during normal working, it is assumed that the LED works at a first drive current to obtain a first received photocurrent of the PD, and the DC offset cancel function is set to a first DC cancel current. The electronic device may obtain the first received photocurrent of the PD through calculation based on a PPG digital signal collected by the AFE, the preset gain of the TIA, and the first DC cancel current. An expression is as follows: The first received photocurrent of the PD is obtained by dividing an ADC input voltage equivalent to the PPG digital signal by the preset gain of the TIA, and then adding the first DC cancel current. The electronic device determines, based on the first received photocurrent of the PD, whether a dimming procedure needs to be enabled. Details are as follows.

Manner 1: In a slot of a cycle, it is detected whether a second PD current exceeds the dimming trigger threshold. If the second PD current exceeds the dimming trigger threshold, the dimming procedure is enabled, so that the second PD current is within the optimal working range of the PD target current. For example, it is determined, in an m^{th} (m=1, 2, 3, or 4) slot (Slot) of an X^{th} PRF cycle, whether a second PD current exceeds the dimming trigger threshold. If the second PD current exceeds the dimming trigger threshold, the dimming procedure is enabled.

Manner 2: In a slot of each cycle or a plurality of cycles, it is detected whether a second PD current exceeds the dimming trigger threshold. If the second PD current exceeds the dimming trigger threshold, the dimming procedure is enabled, so that the second PD current is within the optimal working range of the PD target current. For example, it is determined, in an m^{th} (m=1, 2, 3, or 4) slot (Slot) of an (X+i)^{th} PRF cycle, whether a second PD current exceeds the dimming trigger threshold. If the second PD current exceeds the dimming trigger threshold, the dimming procedure is enabled.

Manner 3: A gain of the TIA of the receive circuit is set to a low gain in a 1^{st} slot or any one or more slots in each PRF cycle. At this gain, the receive circuit (or referred to as a receiving circuit) including the TIA and the ADC is not saturated, or the LED drive current is set to a small value (for example, 5 mA) in this slot. At this current, the receive circuit is not saturated, a CTR in a PRF cycle corresponding to this slot is calculated, and it is deduced, based on the CTR, whether an LED drive current, generated in another slot of a previous PRF cycle or the current PRF cycle, causes the second PD current to exceed the dimming trigger threshold. If the second PD current exceeds the dimming trigger threshold, the dimming procedure is enabled. Alternatively, it is deduced, based on the calculated CTR and PD target current, an LED drive current that needs to be set in the current PRF cycle or the next PRF cycle, and a DC offset current (a DC current output by the IDAC).

In another specific implementation, in one dedicated slot or sub-slot, a CTR is calculated by using a small gain of the TIA or by using a small LED drive current, and it is determined, based on the CTR, whether the dimming procedure needs to be enabled. Details are as follows.

After a CTR value is measured in one slot, it may be determined, based on the CTR measured in the one slot or an LED drive current corresponding to the any one or more other slots, in any one or more of other slots, whether a corresponding second PD current exceeds the dimming trigger threshold. If the second PD current exceeds the dimming trigger threshold, the dimming procedure is enabled. The dimming may include adjusting of light of a same wavelength or color, or adjusting of light of different wavelengths or colors. When each of a plurality of slots corresponds to one wavelength, if a CTR value in one slot is measured, it is deduced, based on a CTR of a corresponding wavelength, whether the dimming procedure needs to be enabled in another slot. In other words, if a 1^{st} slot is a slot in which a CTR is detected, and a 2^{nd} slot is a slot for normally testing the PPG sensor, the dimming procedure may be enabled in a corresponding slot of a current PRF cycle, in other words, detection determining and dimming are performed in a same PRF. For example, it is assumed that different slots correspond to different wavelengths. When the 1^{st} slot emits green light of 530 nm and the 2^{nd} slot emits red light of 660 nm, a CTR of the green light detected in the 1^{st} slot is 80 dB (10000). That is, an LED drive current of the green light is 10000 times a received current of the PD. A CTR of red light can be calculated based on the CTR of the green and a relationship between the CTR of the green light and the CTR of the red light. In this case, it can be deduced, based on the calculated CTR of the red light, whether the dimming procedure needs to be enabled in the 2^{nd} slot. The relationship between the CTR of the green light and the CTR of the red light may be obtained through testing, and may be generally represented by using a formula CTR_red light=k*CTR_green light+b, where k and b are coefficients, and may be obtained through testing. It is assumed that k=0.8 and b=10 are obtained through testing. In this case, it may be calculated, based on the CTR of the green light in the 1^{st} slot, that the CTR of the red light in the 2^{nd} slot is 74, and then it is deduced, based on the CTR (74) of the red light, whether the dimming procedure needs to be enabled in the 2^{nd} slot. It is assumed that different slots correspond to a same wavelength. In this case, it may be calculated, based on the CTR in the 1^{st} slot, a CTR in another slot, and it is determined, based on the CTR of each slot, whether a dimming process needs to be enabled.

Manner 4: Each slot includes two or more sub-slots. A CTR in a corresponding slot is measured in a 1^{st} sub-slot of each slot. It is determined, based on the CTR, whether dimming needs to be performed in a slot of a current PRF cycle or next PRF cycle. That is, dimming in a corresponding slot of the current PRF cycle is enabled, in other words, detection determining and dimming are performed in a same PRF cycle. Alternatively, dimming in a corresponding slot of a next PRF cycle is enabled, in other words, detection determining is performed in a PRF cycle, and dimming is performed in a next PRF cycle.

Manner 5: When a plurality of slots work in each PRF cycle (for example, during a normal PPG test), one or more slots work within the dimming trigger threshold, and a CTR in a corresponding slot may be calculated by using these slots, and it is further deduced whether a CTR and a second PD current that are in another slot exceed the dimming trigger threshold. If the CTR and the second PD current that are in another slot exceed the dimming trigger threshold, a slot where a CTR or second PD current exceeds the dimming trigger threshold is adjusted.

It should be noted that, during dimming adjustment detection, only a CTR of a PD 1 and LED pair ({Pd_ref_i, LEDj}, ref_i, j=1,2, ...) may be detected or calculated, or it may be only detected or calculated whether a receive current of the PD 1 corresponding to an LEDj (j=1, ...) exceeds the dimming trigger threshold.

2. The DC current adjustment may be specifically as follows:
Manner 1: In a slot of a cycle, it is detected whether a difference between a second PD current and a DC current Idac of the DC cancel (where during working, an initial DC cancel current value is set, and the initial DC cancel value may be the PD target current value or a current obtained by adding a value to or subtracting a value from the PD target current value) is within the optimal working range of the TIA, or whether a difference between a second PD current and a first DC current of the DC cancel is within the optimal working range of the TIA. For example, it is detected, in an m^{th} (m=1, 2, 3, or 4) slot (Slot) of an X^{th} PRF cycle, whether a difference between a second PD current and the first DC current of the DC cancel is within the optimal working range of the TIA. If the difference between the second PD current and the first DC current is not within the optimal working range of the TIA, the DC_offset adjustment procedure is enabled. Specifically, the first DC current of the DC Cancel is adjusted to a second DC current, so that the difference between the second PD current and the first DC current of the DC cancel is within the optimal working range of the TIA.
Manner 2: In a slot of each cycle, it is detected whether a difference between a second PD current and a DC current (I_{dac}) of DC cancel is within the optimal working range of the TIA, or it is detected whether a difference between a second PD current and a first DC current of DC cancel is within the optimal working range of the TIA. For example, if the difference between the second PD current and the first DC current is not within the optimal working range of the TIA, the DC_offset adjustment procedure is enabled.
Manner 3: An additional slot or sub-slot is added to detect a CTR in a current PRF cycle (one or more slots or sub-slots may be added for each wavelength, or one or more slots or sub-slots may be added for a plurality of wavelengths). If a change of the CTR exceeds a DC Cancel adjustment trigger threshold, the DC_offset adjustment procedure is enabled. In other words, DC offset adjustment (DC offset cancel adjustment or DC cancel adjustment) may be performed in a subsequent slot or sub-slot of the current PRF cycle.

It should be noted that, during DC_offset adjustment detection, a CTR value of each PD and LED pair ({Pdi, LEDj},I, j=1,2, ...) may be detected or calculated, or it may be detected or calculated whether a received current of each PD corresponding to the LEDj (j=1, ...) exceeds the DC cancel adjustment trigger threshold.

With reference to the foregoing LED dimming and DC current adjustment, the following implementations may exist.

For example, working in a slot is used as an example. When it is detected, in an i^{th} slot (slot) of an N^{th} PRF cycle, that a PD current exceeds a working range (namely, a dimming trigger upper threshold and a dimming trigger lower threshold) of the PD target current, the dimming module calculates in an i^{th} slot of a (N+X₁ (X₁=1, 2, ...))^{th} PRF cycle, a first PD current of the PD 1 based on a first PPG digital signal and a preset gain of the TIA, and obtains a CTR (namely, a current transmission ratio between an LED and a PD) based on a first drive current of the LED and a first PD current. The dimming module may further obtain, based on the CTR and the PD target current, a second drive current that is of the LED and that is needed when the PD target current is reached, and set the LED current to the second drive current in an (i+1)^{th} slot. When it is detected that the difference between the second PD current of the PD 1 and the PD target current is greater than the first threshold when the LED is driven by using the second drive current to emit light, the dimming module controls the DC adjustment module 1 to adjust a DC current, and obtains an adjustment value of the DC current of the DC adjustment module 1. A new DC current is set in an (i+2)^{th} slot, so that a difference between the PD current and the DC current is within the optimal working range of the TIA.

For example, working in a PRF cycle is used as an example. When it is detected, in an N^{th} PRF cycle, that a PD current exceeds a working range (namely, a dimming trigger upper threshold or a dimming trigger lower threshold) of the PD target current, the dimming module calculates, in a (N+X1 (X₁=1, 2, ...))^{th} PRF cycle, a first PD current of the PD 1 based on a first PPG digital signal and a preset gain of the TIA 1, and obtains a CTR (namely, a current transmission ratio between an LED and a PD) based on the first drive current of the LED and the first PD current. The dimming module may further obtain, based on the CTR and the PD target current, a second drive current that is of the LED and that is needed when the PD target current is reached, and set the LED current to the second drive current in an (N+X1+X2 (X2=1,2,...))^{th} PRF cycle. When it is detected that the difference between the second PD current of the PD 1 and the PD target current is greater than the first threshold when the LED is driven by using the second drive current to emit light, the dimming module controls the DC adjustment module 1 to adjust a DC current, and obtains an adjustment value of the DC current of the DC adjustment module 1. A new DC current is set in an (N+X1+X2+X3 (X3=0,1,2,...))^{th} PRF cycle, so that a difference between the PD current and the DC current is within the optimal working range of the TIA.

For example, working in a sub-slot is used as an example. Each PRF cycle is divided into N slots (slots), and each slot is divided into M sub-slots (sub-slots). When it is detected that, in a 1^{st} slot (slot) in a 1^{st} PRF cycle, a PD current exceeds a working range (namely, a dimming trigger upper threshold or a dimming trigger lower threshold) of the PD target current, the dimming module calculates, in an i^{th} sub-slot (sub-slot) of the 1^{st} slot (slot) in the first PRF cycle, a first PD current of the PD based on a first PPG digital signal and a preset gain of the TIA, and obtains a CTR (namely, a current transmission ratio between an LED and a PD) based on the first drive current of the LED and the first PD current. The dimming module may further obtain, based on the CTR and the PD target current, a second drive current that is of the LED and that is needed when the PD target current is reached, and set the LED current to the second drive current in an (i+1)^{th} sub-slot (sub-slot). When it is detected that the difference between the second PD current of the PD and the PD target current is greater than the first threshold when the LED is driven by using the second drive current to emit light, the dimming module controls the DC adjustment module 1 to adjust a DC current, and obtains an adjustment value of the DC current of the DC adjustment module 1. A new DC current is set in an (i+2)^{th} sub-slot (sub-slot), so that a difference between the PD current and the DC current is within the optimal working range of the TIA.

Certainly, LED dimming and DC current adjustment may alternatively be performed during PPG digital signal detection. For a same PD, two receive currents may be used, which are respectively: a low-gain or wide-input receive circuit configured to calculate a CTR; and a high-performance circuit configured to detect a PPG digital signal. For example, when it is detected, in an N^{th} PRF cycle, that a PD current exceeds a working range (namely, a dimming trigger upper threshold or a dimming trigger lower threshold) of the PD target current, the dimming module calculates a first PD current of the PD based on a first PPG digital signal and a preset gain of the TIA, and obtains a CTR (namely, a current transmission ratio between an LED and a PD) based on the first drive current of the LED and the first PD current. The dimming module may further obtain, based on the CTR and the PD target current, a second drive current that is of the LED and that is needed when the PD target current is reached, and set the LED current to the second drive current in an (N+1)^{th} PRF cycle. When it is detected that the difference between the second PD current of the PD and the PD target current is greater than the first threshold when the LED is driven by using the second drive current to emit light, the dimming module controls the DC adjustment module to adjust a DC current, and obtains an adjustment value of the DC current of the DC adjustment module. A new DC current is set in an (N+2)^{th} PRF cycle, so that a difference between the PD current and the DC current is within the optimal working range of the TIA.

It can be learned that LED dimming may be completed in a slot of each cycle or in a sub-slot of each cycle. In this way, in an entire dimming process, dimming duration is greatly shortened, and quick dimming is implemented. Especially, in a rapid fluctuation (for example, violent motion) scenario, the PPG sensor cannot quickly track the PD current. This ensures performance of the receive channel of the AFE chip.

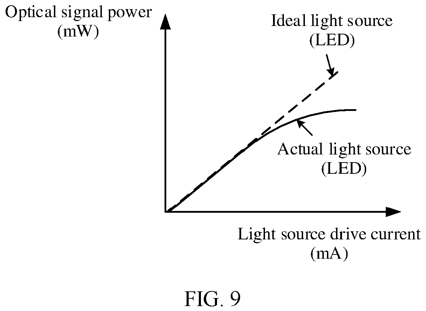

In some other embodiments, the second drive current of the LED may be obtained in another manner. For example, FIG. 9 is a diagram of a correspondence between output optical power of a light source and a drive current of the light source according to an embodiment of this application. As shown in FIG. 9, there is a linear relationship between an output optical power of an ideal light source (for example, an ideal LED) and a drive current of the light source, as shown by a dashed line in FIG. 9. In this case, the dimming module may obtain, based on a CTR, a second drive current that is of the LED and that is needed when the PD target current is reached. However, when the output optical power of the light source and the drive current of the light source are in a non-linear relationship (a solid line shown in FIG. 9), the dimming module may pre-store a correspondence between a current and a power. After the dimming module obtains the CTR, the dimming module may obtain the second drive current of the LED based on the pre-stored correspondence between a current and a power.

For example, a channel loss generated when an optical signal emitted by the light source LED reaches a PD during PPG detection is used as an example for description. In a PPG detection process, the channel loss generated when the optical signal emitted by the LED reaches the PD varies with time. When the LED is driven by using a specified constant drive current to emit light, a change in the channel loss is a root cause that a receive channel exceeds an optimal working range of the AFE chip. Therefore, it is assumed that the channel loss is L=Pₒ/Pᵢ. Pₒ represents a power of the optical signal emitted by the LED, and Pᵢ represents a power of an optical signal received by the PD. Because a linear dynamic range of the PD is high, a PD current and the optical signal received by the PD may be in a linear relationship, that is, I_{pd}=R*Pᵢ. R is a responsivity of the PD, and a unit is A/W or mA/mW.

A relationship between the output optical power of the ideal light source and the drive current of the light source is: Po=k₁+k₂*I_{led}, and the channel loss is: L=Pₒ/Pi=(k₁+k₂*I_{led})*R/I_{pd}=R*(k₁/I_{pd}+k₂*CTR). A relationship between an output optical power of a non-ideal light source and a drive current of the light source is: Pₒ=k₁+k₂*I_{led}+k₃*I_{led}^2, and a channel loss is: L=Pₒ/_{Pi}=R*(k₁/I_{pd}+k₂*CTR+K₃*I_{led} *CTR)=R*(k₁/I_{pd}+k₂*CTR+K₃*I_{pd}*CTR). In this case, the dimming module may obtain a channel loss between the LED and the PD 1 based on the CTR or I_{pd} and I_{led} that are received and detected through current PPG detection or dedicated low-gain configuration, and a relationship between a current and an output optical power that are of the LED. The dimming module may further obtain, based on the PD target current and the channel loss, the second drive current that is of the LED and that is needed when the PD target current is reached. For example, an expression of an output optical power Pₒ' that is of the light source LED and that meets the PD target current is Pₒ'=I_{pd_o}*L/R=I_{pd_o}*L*(k₁/I_{pd}+k2*CTR+K₃*I_{pd}*CTR). I_{pd_o} is the PD target current. The dimming module may further obtain the target drive current of the LED, namely, the second drive current of the light source LED, through calculation or table lookup based on Pₒ' and the relationship between the current and the output optical power that are of the LED.

For example, the relationship between the current and the output optical power that are of the light source LED is a non-linear relationship, that is, P=k₁+k₂*I_{led}+k_{3*}I_{led}^2. In this case, I_{LED_o}={-k₂+sqrt[k₂^2-4*k₃*(k₁-Pₒ')]}/(2*k₃). A table of a drive current of the light source and an output optical power of the light source may be pre-established, and an output optical power Pₒ of the light source may be searched for by using I_{led} as an index. The dimming module may pre-store a correspondence between the drive current of the light source and the output optical power of the light source, and then calculate, based on an I_{pd} current, an optical signal power (Pᵢ=I_{pd}/R) received by the PD 1 and a channel loss (L=Pₒ/Pᵢ) from the light source LED to the PD 1. Then, the dimming module obtains an expected output optical power Pₒ' based on I_{pd_o} and the channel loss from the light source LED to the PD. An expression of Pₒ' is: Pₒ'=L*I_{pd_o}/R, where I_{pd_o} is the PD target current. The dimming module then searches for the pre-established table by using the output optical power Pₒ' as an index, to obtain I_{led_o}, namely, the second drive current of the LED.

In some embodiments, there may be one or more LED drivers (LED drivers). The LED driver may be connected to m LEDs (m≥1) (through a multi-channel analog switch).

In a slot, one or more LED drivers may drive one or more LEDs. For example, in a slot 1 (Slot 1), an LED driver 1 drives IR 1 (an infrared LED 1). In a slot 2 (Slot 2), the LED driver 1 drives IR 1 and IR 2 (the infrared LED 1 and an infrared LED 2). In a slot 3 (Slot 3), the LED driver 1 and the LED driver 2 drive G1 (a green LED 1). In a slot 4 (Slot 4), the LED driver 1 and the LED driver 2 drive G1 (a green LED 1) and G2 (a green LED 2).

In each slot (slot), a configured LED driver and a configured LED may be referred to as one transmit channel. Therefore, there are a plurality of transmit channels in a plurality of slots. In one slot, if there are a plurality of sub-slots, the plurality of sub-slots may still be referred to as a same transmit channel without changing a connection relationship between an LED driver and an LED light. In a plurality of slots, two or more slots may be configured as a same connection relationship between the LED driver and the LED. In this case, the several slots may be referred to as different transmit channels.

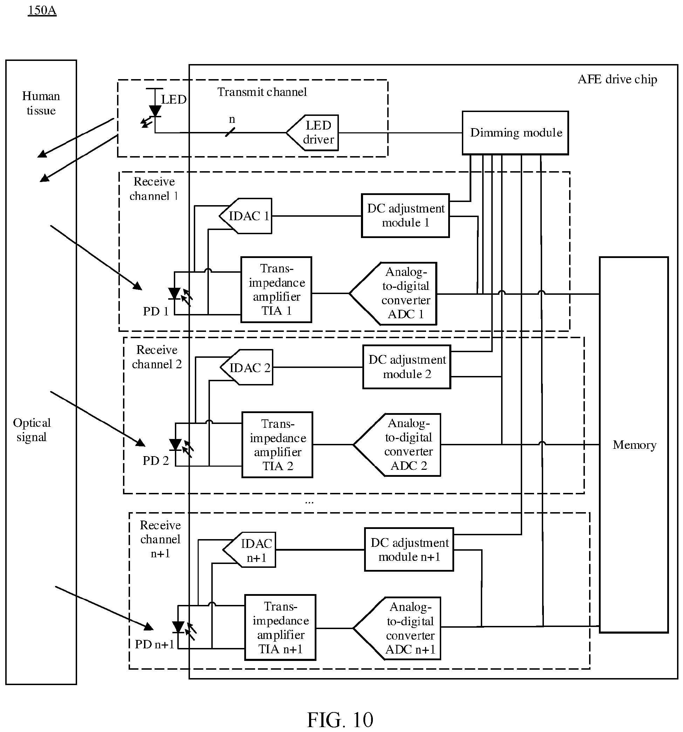

FIG. 10 is a diagram of a structure of a PPG sensor in an electronic device according to an embodiment of this application, to ensure an optical signal receive capability of a PD. The PPG sensor may further include at least one PD, at least one TIA, at least one ADC, at least one DC adjustment module, and at least one IDAC. In other words, there may be one or more TIA trans-impedance amplifiers, namely, J1 (J1≥1) TIA trans-impedance amplifiers. There may be one or more ADCs, namely, J2 (J2≥1) ADCs. There may be one or more IDACs, namely, J3 (J3≥1) IDACs configured to offset a bias current of a PD at an end of a TIA. There may be one or more PDs, namely, J4 (J4≥1) PDs. APD may be connected to any TIA and IDAC, and each TIA may be connected to any ADC. It is assumed that the PD 1, the TIA 1, the ADC 1, the DC adjustment module 1, and the IDAC 1 may form a receive channel 1. In this case, a PD 2, a TIA 2, an ADC 2, a DC adjustment module 2, and an IDAC 2 shown in FIG. 10 form a receive channel 2. As shown in FIG. 10, a PD n+1, a TIA n+1, an ADC n+1, a DC adjustment module n+1, and an IDAC n+1 form a receive channel n+1.

Quantities of receive channels included in all slots may be the same or may be different. A connection relationship between the PD, the IDAC, the TIA, and the ADC in each slot may also be different, and may be randomly combined. For example, in a slot 1 (slot 1), two receive channels (the channel 1 and the channel 2) may be enabled, and a first channel is as follows: The PD 1 and the PD 2 are connected in parallel, then connected to the TIA 1 and the IDAC 1, and then connected to the ADC 2. A second channel is as follows: The PD 1 and a PD 3 are connected in parallel, then connected to the TIA 2 and the IDAC 2, and then connected to an ADC 4.

Automatic dimming refers to adjusting a current of an LED driver of a transmit channel, that is, a drive current of an LED, so that light intensity of an optical signal that is sent by one or more LEDs in a slot and that is returned to the PD after passing through human skin is within an expected range (that is, within a target working current range of the PD). Therefore, when the drive current of the LED is adjusted, one reference receive channel needs to be found, and it is determined whether a photocurrent received by a PD of the reference channel works in the target working current range of the PD based on the photocurrent received by the PD of the reference receive channel. The reference channel may be one receive channel (for example, the receive channel 1 shown in the figure), or may be a plurality of receive channels (for example, the receive channel 1 and the receive channel 2 shown in the figure).

If a plurality of receive channels are used as reference receive channels, it may be determined whether the reference receive channel already works in the target working current range of the PD by using a specified and calculated logic. For example, if it is preset that a plurality of receive channels are used as reference channels in a slot, and the PD target working current is greater than or equal to 2 uA, a drive current (a drive current of a transmit channel) of an LED in the slot needs to be adjusted. Photocurrents received by PDs on all reference receive channels are greater than or equal to 2 uA.

For example, it is preset that a plurality of receive channels are used as reference channels in a slot, and a PD target working current range of the plurality of receive channels is a range: [10 uA, 20 uA], of sum of photocurrents received by PDs of the plurality of reference receive channels. In this case, a drive current (a drive current of a transmit channel) of an LED in the slot needs to be adjusted to ensure that a sum of photocurrents received by PDs of all reference receive channels is between 10 uA and 20 uA.

If one receive channel is used as a reference receive channel, for example, the receive channel 1, only a PD of the receive channel 1 needs to work in a specified target working current range of the PD during dimming.

In general, for a manner of adjusting a DC current of the DC adjustment module on each receive channel, refer to the foregoing manner of adjusting the DC current on the receive channel 1. Details are not described herein again. Optionally, the receive channels in the electronic device shown in FIG. 10 may work concurrently. In other words, when the receive channel 1 performs DC current adjustment, at least one other receive channel may also perform DC current adjustment.

To have a capability of storing a PPG digital signal, the PPG sensor 150A further includes a memory. The memory is connected to at least one ADC, and is configured to: receive and store a PPG digital signal output by the at least one ADC. For example, as shown in FIG. 2, the memory is configured to store a PPG digital signal output by the ADC 1. As shown in FIG. 10, the memory is configured to store a PPG digital signal output by the ADC 1, a PPG digital signal output by an ADC 2, and a PPG digital signal output by an ADC n+1.

Optionally, in some embodiments of this application, the electronic device may be, for example, a wearable device. In embodiments of this application, a light source dimming method is provided. To save resources, a working status detection module and a working mode switching module are disposed in the wearable device. When the working status detection module detects that an optical signal emitted by an LED of a PPG sensor is returned to a PD without being transmitted or reflected, it is determined that the wearable device does not perform the detection operation, that is, the wearable device is in a rest state. In this case, the working mode switching module switches the wearable device to a standby mode or a power-saving mode. When the working status detection module detects that an optical signal emitted by an LED of the PPG sensor is returned to the PD through transmission or reflection, it is determined that the wearable device performs working. In this case, the working mode switching module switches the wearable device to a working mode.

For example, the wearable device in embodiments of this application may be a wired headset, a wireless headset (for example, a TWS Bluetooth headset, a neckband Bluetooth headset, or a head-mounted Bluetooth headset), a smartwatch, a smart band, smart glasses, a smart anklet, a smart ring, a smart necklace, an augmented reality (augmented reality, AR) device, a virtual reality (virtual reality, VR) device, and the like. A specific form of the wearable device is not specially limited in this application. Optionally, in this embodiment of this application, an example in which the wearable device is a smartwatch is used. The smartwatch can further implement sleep detection, heart rate detection, and the like.

Optionally, in some other embodiments of this application, the electronic device may be a non-wearable device. For example, the non-wearable device may be a medical fingertip oximeter, a facial recognition device (for example, a facial recognition access control device or a terminal device with facial recognition), or a device with a fingerprint unlocking function (for example, a fingerprint clocking machine or a mobile device with a fingerprint unlocking function).

Technical solutions in the following embodiments may be implemented in hardware structures shown in FIG. 7A, FIG. 7B, and FIG. 10.

A light source dimming method provided in embodiments of this application is applicable to an electronic device, a wearable device, and the like mentioned in embodiments of this application. An electronic device is used as an example. The electronic device may be of a structure shown in FIG. 7A and FIG. 7B. The light source dimming method may include three dimming manners (the following manner 1, manner 2, and manner 6), and three DC_offset adjustment manners (the following manner 3, manner 4, and manner 5). Specifically, the manner 1, manner 2, manner 3, manner 4, manner 5, and manner 6 are separately described in detail. Details are as follows.

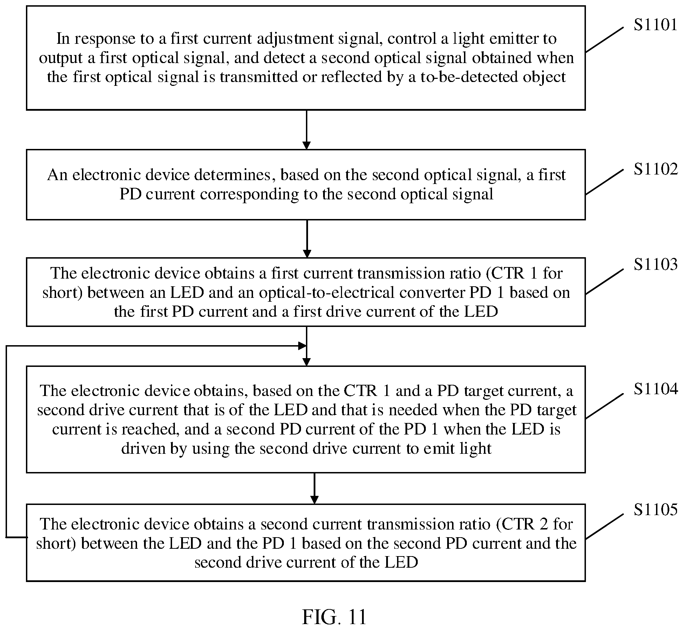

Manner 1: FIG. 11 is a schematic flowchart of implementing a light source dimming method according to an embodiment of this application. As shown in FIG. 11, details are as follows.

S1101: In response to a first current adjustment signal, an electronic device controls a light emitter to output a first optical signal, and detect a second optical signal obtained when the first optical signal is transmitted or reflected by a to-be-detected object.

The electronic device presets a TIA 1 of a PPG sensor to a small gain, and sets a first drive current (for example, the first electrical signal) of an LED of the PPG sensor in an i^{th} PRF cycle. The light emitter (for example, the LED) is driven by using the first drive current to output the first optical signal.

The electronic device may output the first optical signal through the light emitter (for example, the LED) of the PPG sensor, and a PD 1 of the PPG sensor collects a second optical signal. The second optical signal is obtained when the first optical signal is transmitted or reflected by the to-be-detected object.

S1102: The electronic device determines, based on the second optical signal, a first photocurrent corresponding to the second optical signal.

Specifically, the electronic device determines, based on the second optical signal, an electrical signal corresponding to the second optical signal. The electrical signal may be a PPG digital signal output by an ADC 1 of the PPG sensor. The PPG digital signal is a signal that is output after the second optical signal collected by the PD 1 passes through the TIA 1 and the ADC 1. Specifically, the electronic device obtains a first PPG digital signal from the ADC 1. The electronic device determines a first PD current (a first photocurrent) of the PD 1 based on the PPG digital signal.

S1103: The electronic device obtains a first current transmission ratio (CTR 1 for short) between the LED and the PD 1 based on the first PD current and the first drive current of the LED.

An expression of the CTR is: CTR=first drive current of the LED/first PD current corresponding to the second optical signal.

Specifically, the electronic device calculates the first PD current of the PD 1 based on the first PPG digital signal and a preset gain of the TIA 1. The electronic device may obtain the first current transmission ratio between the LED and the PD 1 based on the first drive current of the LED and the first PD current.

S1104: The electronic device determines, based on the CTR 1 and a PD target current, a second drive current of the LED and a second PD current of the PD when the LED is driven by using the second drive current to emit light.

In an implementation, the electronic device may obtain, based on the first current transmission ratio between the LED and the PD 1, and the PD target current, the second drive current that is of the LED and that is needed when the PD target current is reached, and the second PD current of the PD 1 when the LED is driven by using the second drive current to emit light.

In another implementation, the electronic device may pre-store a correspondence between a drive current and a power. After the electronic device obtains the CTR, the electronic device may obtain the second drive current of the LED based on the pre-stored correspondence between a current and a power.

For example, a relationship between a current and an output optical power that are of the light source LED is a non-linear relationship, that is, P=k₁+k₂*I_{led}+k₃*I_{led}^2. In this case, I_{LED_o}={-k₂+sqrt[k₂^2-4*k₃*(k₁-Pₒ')]}/(2*k₃). A table of a drive current of the light source and an output optical power of the light source may be pre-established, and an output optical power Pₒ of the light source may be searched for by using I_{led} as an index. The electronic device may pre-store a correspondence between the drive current of the light source and the output optical power of the light source, and then calculate, based on an I_{pd} current, an optical signal power (Pi=I_{pd}/R) received by the PD and a channel loss (L=Pₒ/Pᵢ) from the light source LED to the PD. Then, the electronic device obtains an expected output optical power Pₒ' based on I_{pd_o} and the channel loss from the light source LED to the PD. An expression of Pₒ' is: Pₒ'=L*I_{pd_o}/R, where I_{pd_o} is the PD target current. Further, the electronic device searches for the pre-established table by using the output optical power Pₒ' as an index, to obtain I_{led_o}, namely, the second drive current of the LED.

S1105: The electronic device obtains a second current transmission ratio (CTR 2 for short) between the LED and the PD 1 based on the second photocurrent and the second drive current of the LED.

An implementation of this step is the same as an implementation of step S1103. For specific implementation, refer to the description in step S1103. Details are not described herein again.

In this way, steps S1104 and S1105 may be repeatedly performed to continue to obtain, based on the second current transmission ratio between the LED and the PD 1 and the PD target current, a third drive current that is of the LED and that is needed when the PD target current is reached, and a third PD current of the PD 1 when the LED is driven by using the third drive current to emit light, until an N^{th} (N=1, 2, ...) PD current is within an optimal working range of the PD target current.

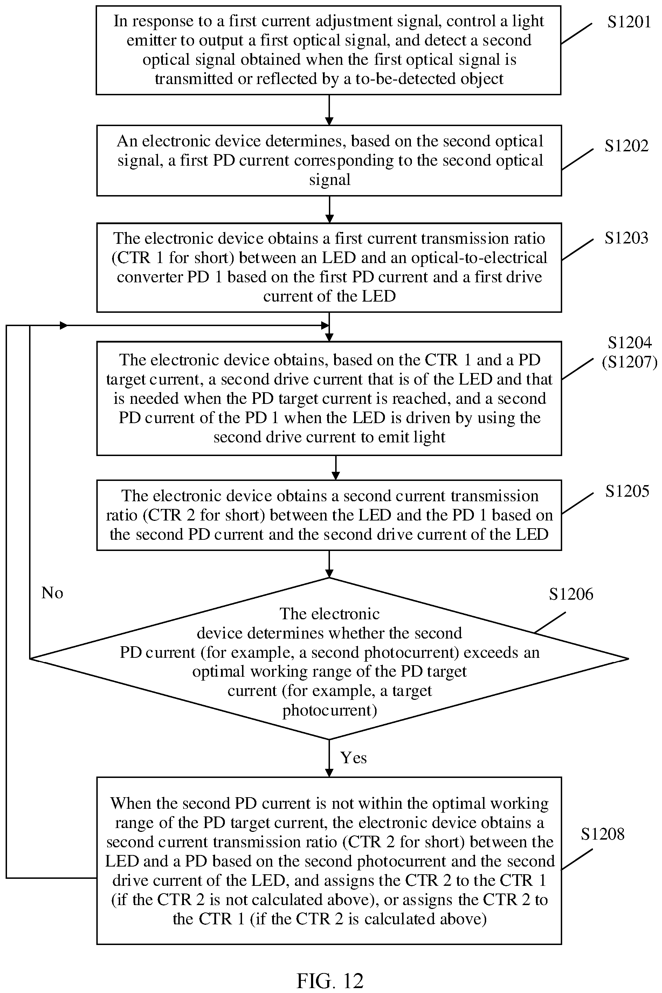

Manner 2: FIG. 12 is another schematic flowchart of implementing a light source dimming method according to an embodiment of this application. As shown in FIG. 12, details are as follows.

S1201: In response to a first current adjustment signal, an electronic device controls a light emitter to output a first optical signal, and detect a second optical signal obtained when the first optical signal is transmitted or reflected by a to-be-detected object.

An implementation of this step is the same as an implementation of step S1101. For specific implementation, refer to the description in step S1101. Details are not described herein again.

S1202: The electronic device determines, based on the second optical signal, a first photocurrent corresponding to the second optical signal.

An implementation of this step is the same as an implementation of step S1102. For specific implementation, refer to the description in step S1102. Details are not described herein again.

S1203: The electronic device obtains a first current transmission ratio (CTR 1 for short) between an LED and a PD 1 based on the first photocurrent and a first drive current of the LED.

An implementation of this step is the same as an implementation of step S1103. For specific implementation, refer to the description in step S1103. Details are not described herein again.

S1204: The electronic device determines, based on the CTR 1 and a PD target current, a second drive current of the LED and a second PD current of a PD when the LED is driven by using the second drive current to emit light.

An implementation of this step is the same as an implementation of step S1104. For specific implementation, refer to the description in step S1104. Details are not described herein again.

S1205: The electronic device obtains a second current transmission ratio (CTR 2 for short) between the LED and the PD 1 based on the second photocurrent and the second drive current of the LED.

An implementation of this step is the same as an implementation of step S1105. For specific implementation, refer to the description in step S1105. Details are not described herein again.

S1206: The electronic device determines whether the second PD current (for example, the second photocurrent) exceeds an optimal working range of the PD target current (for example, the target photocurrent). If the second PD current does not exceed the optimal working range of the PD target current, step S1204 is performed; or if the second PD current exceeds the optimal working range of the PD target current, step S1207 is performed.

Specifically, when a difference between the second PD current and the PD target current is greater than a first threshold, it is determined that the second PD current exceeds the optimal working range of the PD target current; or when a difference between the second PD current and the PD target current is less than or equal to a first threshold, it is determined that the second PD current is within the optimal working range of the PD target current. The first threshold may be a DC adjustment trigger threshold.

In a specific implementation, an occasion for performing step S1206 may include: in an m^{th} slot of an X^{th} cycle; or in an m^{th} slot of each cycle. In another specific implementation, step S1206 may be specifically performed as follows: After the current transmission ratio is measured in a 1^{st} slot, determining, in each of other slots based on a third drive current generated in the 1^{st} slot, whether a photocurrent corresponding to the third drive current is within the optimal working range of the target photocurrent; or after the current transmission ratio is measured in a 1^{st} sub-slot of each slot, determining, based on the current transmission ratio in a slot of a first cycle to which the 1^{st} sub-slot belongs or a slot of a second cycle to which the 1^{st} sub-slot belongs, whether the second photocurrent is within the optimal working range of the target current; or when working is performed in the one or more slots of each cycle within a dimming trigger threshold, determining the current transmission ratio, and determining whether a current transmission ratio in another slot and the second photocurrent are in the optimal working range of the target current.

S1207: When the second PD current is within the optimal working range of the PD target current, the electronic device drives the LED to emit light by using the second drive current.

Optionally, step S1208, when the second PD current is not within the optimal working range of the PD target current, the electronic device obtains a second current transmission ratio (CTR 2 for short) between the LED and the PD based on the second photocurrent and the second drive current of the LED, and assigns the CTR 2 to the CTR 1 (if the CTR 2 is not calculated above), or assigns the CTR 2 to the CTR 1 (if the CTR 2 is calculated above).

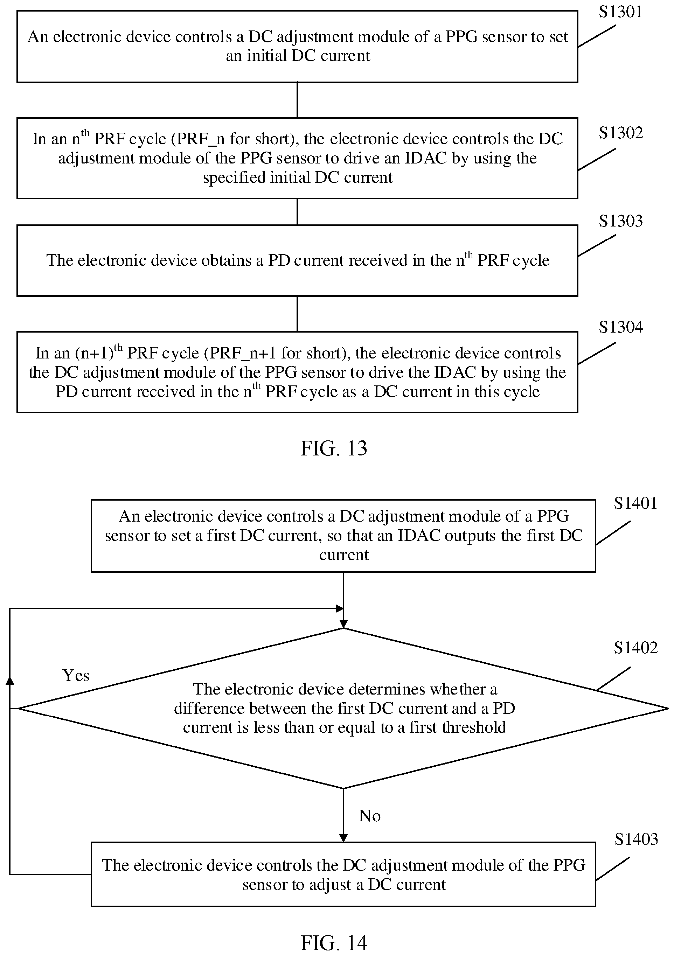

Manner 3: FIG. 13 is another schematic flowchart of implementing a light source dimming method or a DC current adjustment method according to an embodiment of this application. As shown in FIG. 13, details are as follows.

S1301: An electronic device controls a DC adjustment module of a PPG sensor to set an initial DC current.

The initial DC current may be a PD target current or a current of a PD target current value +/- a value. The initial DC current may be represented as I_{DC_O_1}.

S1302: In an n^{th} PRF cycle (PRF_n for short), the electronic device controls the DC adjustment module of the PPG sensor to drive an IDAC by using the specified initial DC current.

S1303: The electronic device obtains a PD current received in the n^{th} PRF cycle.

S1304: In an (n+1)^{th} PRF cycle (PRF_n+1 for short), the electronic device controls the DC adjustment module of the PPG sensor to drive the IDAC by using the PD current received in the (n+1)^{th} PRF cycle as a DC current in this cycle.

For example, a plurality of PRF cycles are used as a basis of a calculation operation. For example, it is assumed that 10 PRF cycles are used as a basis, the IDAC is set to work at a first DC current value in the 1^{st} to 10^{th} PRF cycles. PD current values in the 1^{st} to 10^{th} PRF cycles are calculated to obtain an average value. In 11^{th} to 20^{th} PRF cycles, the average value of the PD current values in the 1^{st} to 10^{th} PRF cycles is used as a DC current value for working. In this way, the DC current can be adjusted more stably.

Manner 4: FIG. 14 is another schematic flowchart of implementing a light source dimming method or a DC current adjustment method according to an embodiment of this application. As shown in FIG. 14, details are as follows.

S1401: An electronic device controls a DC adjustment module of a PPG sensor to set a first DC current, so that an IDAC outputs a first DC current. The first DC current may be represented as I_{DC_O}.

S1402: The electronic device determines whether a difference between the first DC current and a PD current is less than or equal to a first threshold. An expression of the first threshold may be: I_{PD_2-}I_{DC_O}|≤ε. If the difference between the first DC current and the PD current is less than or equal to the first threshold, step S1401 is performed. If the difference between the first DC current and the PD current is greater than the first threshold, step S1403 is performed.

There may be two implementations in which the electronic device determines the difference between the first DC current and the PD current. Details are as follows.

Implementation 1: The electronic device may determine, in any slot of a current PRF cycle (a same PRF cycle) in a current PRF cycle, the difference between the first DC current and the PD current, and then set any DC current value in the current PRF cycle (the same PRF cycle), that is, determine the difference in a prediction manner, and set the DC current value in advance.

Implementation 2: Within or after one PRF cycle, the electronic device calculates, in any slot of a current PRF cycle, the difference between the PD current and the first DC current, and determines a DC current value in any slot of a next PRF cycle.

S1403: The electronic device controls the DC adjustment module of the PPG sensor to adjust a DC current.

Specifically, the electronic device calculates a second DC current, that is, the IDAC outputs the second DC current. For example, a calculation expression is: ΔI_{dc}=I_{PD_2}-I_{DC_O_1}, new I_{DC_O_2}=I_{DC_O_1}+ΔI_{dc}=I_{PD_2}, and new I_{DC_O_2} is assigned to I_{DC_O}.

In this way, steps S1402 and S1403 may be repeatedly performed. When the difference between the first DC current and the PD current is less than or equal to the first threshold, a new DC current obtained through calculation is continuously assigned to the first DC current.

For example, a plurality of PRF cycles are used as a basis of a calculation operation. For example, it is assumed that 10 PRF cycles are used as a basis, the IDAC is set to work at a first DC current value in the 1^{st} to 10^{th} PRF cycles. PD currents in the 1^{st} to 10^{th} PRF cycles are calculated to obtain a difference between an average value and the DC current, and a new DC current value. In 11^{th} to 20^{th} PRF cycles, working is performed at the new DC current value in the 1^{st} to 10^{th} PRF cycles. In this way, the DC current can be adjusted more stably.

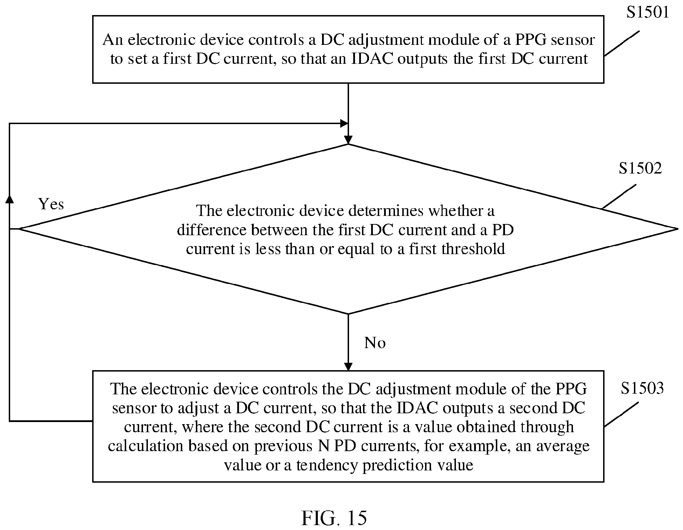

Manner 5: FIG. 15 is another schematic flowchart of implementing a light source dimming method or a DC current adjustment method according to an embodiment of this application. As shown in FIG. 15, details are as follows.

S1501: An electronic device controls a DC adjustment module of a PPG sensor to set a first DC current, so that an IDAC outputs a first DC current. The first DC current may be represented as I_{DC_O}.

S1502: The electronic device determines whether a difference between the first DC current and a PD current is less than or equal to a first threshold. An expression of the first threshold may be: |I_{PD_2-}I_{DC_O}|≤ε. If the difference between the first DC current and the PD current is less than or equal to the first threshold, step S1501 is performed. If the difference between the first DC current and the PD current is greater than the first threshold, step S1503 is performed.

S1503: The electronic device controls the DC adjustment module of the PPG sensor to adjust a DC current, so that the IDAC outputs a second DC current, where the second DC current is a value obtained through calculation based on previous N (N=0, 1, 2, ...) PD currents, for example, an average value or a tendency prediction value.

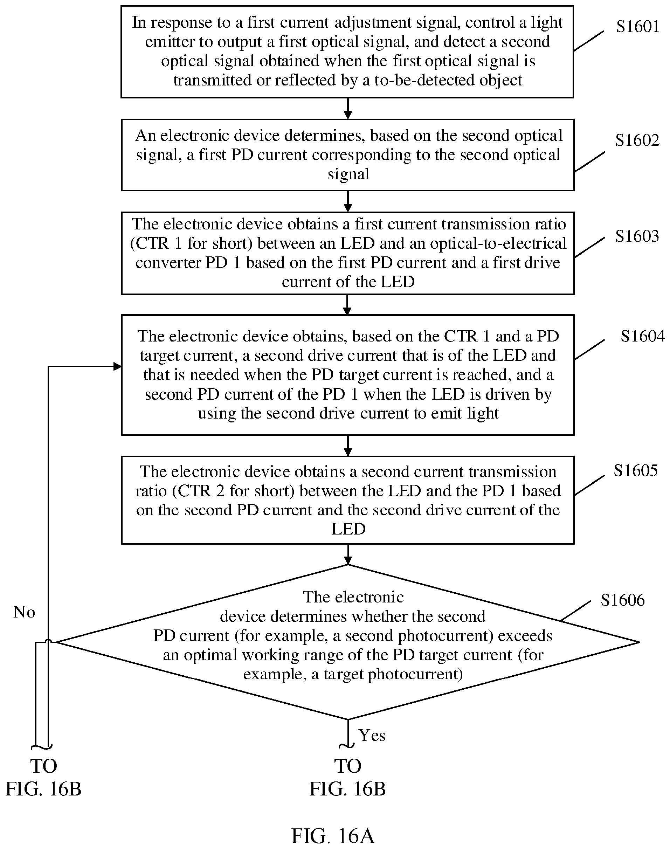

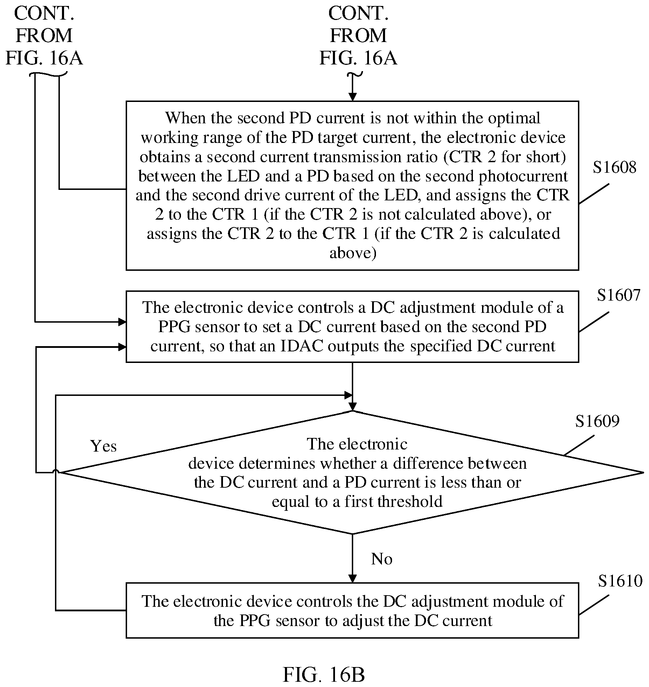

Manner 6: FIG. 16A and FIG. 16B are still another schematic flowchart of implementing a light source dimming method according to an embodiment of this application. As shown in FIG. 16A and FIG. 16B, details are as follows.

S1601: In response to a first current adjustment signal, an electronic device controls a light emitter to output a first optical signal, and detects a second optical signal obtained when the first optical signal is transmitted or reflected by a to-be-detected object.

An implementation of this step is the same as an implementation of step S1101. For specific implementation, refer to the description in step S1101. Details are not described herein again.

S1602: The electronic device determines, based on the second optical signal, a first photocurrent corresponding to the second optical signal.

An implementation of this step is the same as an implementation of step S1102. For specific implementation, refer to the description in step S1102. Details are not described herein again.

S1603: The electronic device obtains a first current transmission ratio (CTR 1 for short) between an LED and a PD 1 based on the first photocurrent and a first drive current of the LED.

An implementation of this step is the same as an implementation of step S1103. For specific implementation, refer to the description in step S1103. Details are not described herein again.

S1604: The electronic device determines, based on the CTR 1, a second drive current of the LED and a second PD current of a PD when the LED is driven by using the second drive current to emit light.

An implementation of this step is the same as an implementation of step S1104. For specific implementation, refer to the description in step S1104. Details are not described herein again.

S1605: The electronic device obtains a second current transmission ratio (CTR 2 for short) between the LED and the PD 1 based on the second photocurrent and the second drive current of the LED.

An implementation of this step is the same as an implementation of step S1105. For specific implementation, refer to the description in step S1105. Details are not described herein again.

S1606: The electronic device determines whether the second PD current (for example, the second photocurrent) exceeds an optimal working range of a PD target current (for example, the target photocurrent). If the second PD current does not exceed the optimal working range of the PD target current, step S1607 is performed; or if the second PD current exceeds the optimal working range of the PD target current, step S1604 is performed.

Optionally, step S1608, when the second PD current is not within the optimal working range of the PD target current, the electronic device obtains a second current transmission ratio (CTR 2 for short) between the LED and the PD based on the second photocurrent and the second drive current of the LED, and assigns the CTR 2 to the CTR 1 (if the CTR 2 is not calculated above), or assigns the CTR 2 to the CTR 1 (if the CTR 2 is calculated above).

S1607: The electronic device controls a DC adjustment module of a PPG sensor to set a DC current based on the second PD current, so that an IDAC outputs the specified DC current.

Specifically, after the electronic device controls the DC adjustment module of the PPG sensor to adjust the DC current, the electronic device obtains an adjustment value of the DC current of the DC adjustment module. There is a correspondence among the adjustment value, the second PD current, the PD target current, and a convergence threshold of an optimal working range of a receiver circuit of an AFE chip. For example, an expression of the correspondence may be |I_{PD_2}-(I_{PD_O}+Δ_{dc})≤c, and c≤a. I_{PD_2} is the second PD current; I_{PD_O} is the PD target current; ΔI_{dc} is the adjustment value of I_{dc}; an initial value of I_{dc} may be set to I_{PD_O}; c is the coverage threshold of the optimal working range of the PD target current; and a is a DC adjustment trigger threshold. In this way, the electronic device may obtain a range of a current input to the TIA (the range is related to the DC adjustment trigger threshold) and a range of a voltage signal output by the TIA. The voltage signal is obtained by multiplying a gain configured for the TIA by a current range of the TIA. Therefore, the gain of the TIA is set, so that the TIA and the ADC of the receive channel of the AFE chip can work within an optimal working range.

S1609: The electronic device determines whether a difference between the DC current and a PD current is less than or equal to a first threshold. An expression of the first threshold may be: |I_{PD_2-}I_{DC_O}|≤ε. If the difference between the first DC current and the PD current is less than or equal to the first threshold, step S1607 is performed. If the difference between the first DC current and the PD current is greater than the first threshold, step S1610 is performed.

There may be two implementations in which the electronic device determines the difference between the first DC current and the PD current. Details are as follows.

Implementation 1: The electronic device may determine the difference in any slot of a current PRF cycle (a same PRF cycle) in a current PRF cycle, and then set any DC current value in the current PRF cycle (the same PRF cycle), that is, determine the difference in a prediction manner, and set the DC current value in advance.

Implementation 2: Within or after one PRF cycle, the electronic device calculates, in any slot of a current PRF cycle, the difference between the PD current and the first DC current, and determines a DC current value in any slot of a next PRF cycle.

In a specific implementation, step S1609 is specifically: determining in an n^{th} slot of an X^{th} cycle, whether a difference between the second PD current and a DC current of a DC Cancel is within an optimal working range of a TIA, where both X and n are positive integers greater than or equal to 1; or determining in an n^{th} slot of each cycle, whether a difference between the second PD current and a DC current of a DC Cancel is within an optimal working range of a TIA.

S1610: The electronic device controls the DC adjustment module of the PPG sensor to adjust the DC current.

Specifically, the electronic device calculates a second DC current, that is, the IDAC outputs the second DC current. For example, a calculation expression is: ΔI_{dc}=I_{PD_2}-I_{DC_O_1}, new I_{DC_O_2}=I_{DC_O_1}+ΔI_{dc}=I_{PD_2}, and new I_{DC_O_2} is assigned to I_{DC_O}.

In this way, steps S1402 and S1403 may be repeatedly performed. When the difference between the first DC current and the PD current is less than or equal to the first threshold, a new DC current obtained through calculation is continuously assigned to the first DC current.

For example, a plurality of PRF cycles are used as a basis of a calculation operation. For example, it is assumed that 10 PRF cycles are used as a basis, the IDAC is set to work at a first DC current value in the 1^{st} to 10^{th} PRF cycles. PD currents in the 1^{st} to 10^{th} PRF cycles are calculated to obtain a difference between an average value and the DC current, and a new DC current value. In 11^{th} to 20^{th} PRF cycles, working is performed at the new DC current value in the 1^{st} to 10^{th} PRF cycles. In this way, the DC current can be adjusted more stably.

In some other embodiments, the electronic device is the electronic device shown in FIG. 10. A difference from the electronic device shown in FIG. 7A and FIG. 7B lies in the following:
Before step S1101, the method further includes:
S1: In response to a received current adjustment signal, an electronic device traverses PPG data channels corresponding to an AFE chip of a PPG sensor, to determine a PPG data channel corresponding to an i^{th} time period.

The PPG data channels corresponding to the AFE chip may be N PPG data channels preconfigured for the AFE chip, where N is a positive integer greater than 1.

Data of a plurality of data channels may be collected in one sampling period. For example, one data channel corresponds to one time period in one sampling period. One PPG data channel is used to control an LED of one PPG sensor and a corresponding PD.

That the electronic device traverses PPG data channels corresponding to an AFE chip of a PPG sensor may be understood as that the electronic device traverses the PPG data channels in a sequence of sequence numbers of the PPG data channels. For example, traversal starts from a first PPG data channel, and ends at an N^{th} PPG data channel.

Optionally, the PPG data channel may be a channel corresponding to a PD (a photodetector), that is, each PD detector corresponds to one data channel. Alternatively, the PPG data channel may be a channel corresponding to a TIA (a TIA receive channel), that is, each TIA receive channel corresponds to one data channel. Alternatively, the PPG data channel may be a channel corresponding to any combination of a slot, a PD detector, and a TIA (a TIA receive channel), that is, any combination of the slot, the PD detector, and the TIA(the TIAreceive channel) corresponds to one data channel. Each PPG data channel may correspondingly output one PPG data signal.

S2: The electronic device determines a PPG digital signal of the PPG data channel corresponding to the i^{th} time period.

An embodiment of this application further provides a chip system. The chip system includes at least one processor and at least one interface circuit. The processor and the interface circuit may be interconnected through a line. For example, the interface circuit may be configured to receive a signal from another apparatus (for example, a memory). For another example, the interface circuit may be configured to send a signal to another apparatus (for example, a processor). For example, the interface circuit may read instructions stored in the memory, and send the instructions to the processor. When the instructions are executed by the processor, the electronic device is enabled to perform the steps performed by the wearable device in the foregoing embodiments. Certainly, the chip system may further include another discrete device. This is not specifically limited in embodiments of this application.

An embodiment of this application further provides an apparatus. The apparatus is included in an electronic device, and the apparatus has a function of implementing behavior of the electronic device in any method in the foregoing embodiments. The function may be implemented by hardware, or may be implemented by hardware executing corresponding software. The hardware or the software includes at least one module or unit corresponding to the foregoing functions, for example, a detection module or unit and a determining module or unit.

An embodiment of this application further provides a computer-readable storage medium, including computer instructions. When the computer instructions are run on an electronic device, the electronic device is enabled to perform any method in the foregoing embodiments.

An embodiment of this application further provides a computer program product. When the computer program product runs on a computer, the computer is enabled to perform any method in the foregoing embodiments.

It may be understood that to implement the foregoing functions, the terminal includes corresponding hardware structures and/or corresponding software modules for performing the functions. A person skilled in the art should easily be aware that, in combination with units and algorithm steps of the examples described in embodiments disclosed in this specification, embodiments of this application may be implemented by hardware or a combination of hardware and computer software. Whether a function is performed by hardware or hardware driven by computer software depends on particular applications and design constraints of the technical solutions. A person skilled in the art may use different methods to implement the described functions for each particular application, but it should not be considered that the implementation goes beyond the scope of embodiments of the present invention.

In embodiments of this application, the terminal or the like may be divided into functional modules based on the foregoing method examples. For example, each functional module may be obtained through division based on each corresponding function, or two or more functions may be integrated into one processing module. The integrated module may be implemented in a form of hardware, or may be implemented in a form of a software functional module. It should be noted that, in embodiments of the present invention, module division is an example, and is merely a logical function division. During actual implementation, another division manner may be used.

The foregoing descriptions about implementations allow a person skilled in the art to understand that, for the purpose of convenient and brief description, division of the foregoing functional modules is taken as an example for illustration. During actual application, the foregoing functions can be allocated to different functional modules and implemented based on a requirement, that is, an inner structure of an apparatus is divided into different functional modules to implement all or some of the functions described above. For detailed working processes of the foregoing system, apparatus, and unit, refer to corresponding processes in the foregoing method embodiments. Details are not described herein again.

Functional units in each of embodiments of this application may be integrated into one processing unit, or each of the units may exist alone physically, or two or more units are integrated into one unit. The integrated unit may be implemented in a form of hardware, or may be implemented in a form of a software functional unit.

When the integrated unit is implemented in the form of the software functional unit and sold or used as an independent product, the integrated unit may be stored in a computer-readable storage medium. Based on such an understanding, the technical solutions of embodiments of this application essentially, or the part contributing to the conventional technology, or all or some of the technical solutions may be implemented in a form of a software product. The computer software product is stored in a storage medium and includes several instructions for instructing a computer device (which may be a personal computer, a server, or a network device) or a processor to perform all or some of the steps of the methods described in embodiments of this application. The foregoing storage medium includes any medium that can store program code, such as a flash memory, a removable hard disk, a read-only memory, a random access memory, a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. An electronic device, wherein the electronic device comprises a first light emitter, a first optical-to-electrical converter, a first trans-impedance amplifier, a first analog-to-digital converter, a dimming module, and a first DC adjustment module, wherein
the other end of the first analog-to-digital converter is electrically connected to one end of the dimming module, the other end of the dimming module is electrically connected to the first light emitter and one end of the first DC adjustment module, the other end of the first DC adjustment module is electrically connected to one end of the first optical-to-electrical converter, the other end of the first optical-to-electrical converter is electrically connected to one end of the first trans-impedance amplifier, and the other end of the first trans-impedance amplifier is electrically connected to one end of the first analog-to-digital converter; and
the dimming module is configured to: determine a first photocurrent of the first optical-to-electrical converter based on a digital signal output by the first analog-to-digital converter and a preset gain of the first trans-impedance amplifier; determine a current transmission ratio between the first light emitter and the first optical-to-electrical converter based on the first photocurrent and a first drive current of the first light emitter; determine, based on the current transmission ratio, a second drive current of the first light emitter, and a second photocurrent of the first optical-to-electrical converter when the first light emitter is driven by using the second drive current to emit light; and when it is determined that the second photocurrent is within an optimal working range of a target current, adjust a DC current of the first DC adjustment module based on the second photocurrent, for a difference between the second photocurrent and the DC current to be within an optimal working range of the first trans-impedance amplifier in a high-gain mode.

2. The electronic device according to claim 1, wherein the dimming module is configured to:
determine, based on the current transmission ratio and a target photocurrent, the second drive current that is of the first light emitter and that is needed when the target photocurrent is reached; or
determine, based on the current transmission ratio and a pre-stored correspondence between a drive current and a power, the second drive current of the first light emitter.

3. The electronic device according to claim 1 or 2, wherein the dimming module is further configured to:
when a difference between the second photocurrent and the target photocurrent is greater than a first threshold, control the first DC adjustment module to adjust the DC current, and obtain an adjustment value of the DC current of the first DC adjustment module, wherein there is a correspondence among the adjustment value, the second photocurrent, the target photocurrent, and a convergence threshold of the optimal working range of the target photocurrent, and the first threshold is a DC adjustment trigger threshold.

4. The electronic device according to claim 1 or 2, wherein the dimming module is further configured to:
when a difference between the second photocurrent and the DC current of the first DC adjustment module is greater than a second threshold, control the first DC adjustment module to adjust the DC current, and obtain an adjustment value of the DC current of the first DC adjustment module, wherein there is a correspondence among the adjustment value, the second photocurrent, the DC current of the first DC adjustment module, and a convergence threshold of the optimal working range of the target photocurrent.

5. The electronic device according to any one of claims 1 to 4, wherein the dimming module is further configured to:
determine, in an m^{th} slot of an X^{th} cycle, that the second photocurrent exceeds the optimal working range of the target current, wherein both X and m are positive integers greater than or equal to 1; or
determine, in an m^{th} slot of each cycle, that the second photocurrent exceeds the optimal working range of the target photocurrent; or
after the current transmission ratio is measured in a 1^{st} slot, determine, in another slot based on a third drive current generated in the 1^{st} slot, that a photocurrent corresponding to the third drive current exceeds the optimal working range of the target photocurrent; or
after the current transmission ratio is measured in a 1^{st} sub-slot of each slot, determine, based on the current transmission ratio and in a slot of a first cycle to which the 1^{st} sub-slot belongs or a slot of a second cycle to which the 1^{st} sub-slot belongs, that the second photocurrent exceeds the optimal working range of the target current; or
when working is performed in one or more slots of each cycle within a dimming trigger threshold, determine the current transmission ratio, and determine that a current transmission ratio and a second photocurrent that are in another slot exceed the optimal working range of the target current.

6. The electronic device according to any one of claims 1 to 5, wherein the dimming module is further configured to:
determine, in an n^{th} slot of the X^{th} cycle, that the difference between the second photocurrent and the DC current exceeds an optimal working range of a TIA, wherein both X and n are positive integers greater than or equal to 1; or
determine, in an n^{th} slot of each cycle, that the difference between the second photocurrent and the DC current exceeds an optimal working range of a TIA.

7. The electronic device according to any one of claims 1 to 6, wherein the electronic device further comprises a drive chip, wherein the drive chip comprises a first driver, the first trans-impedance amplifier, the first analog-to-digital converter, the dimming module, the first DC adjustment module, and a first current digital-to-analog converter, wherein
the other end of the dimming module is electrically connected to one end of the first driver, the other end of the first driver is electrically connected to the first light emitter, the other end of the first DC adjustment module is electrically connected to one end of the first current digital-to-analog converter, another end of the first current digital-to-analog converter is electrically connected to one end of the first optical-to-electrical converter, and the other end of the first optical-to-electrical converter is electrically connected to still another end of the first current digital-to-analog converter.

8. The electronic device according to any one of claims 1 to 7, wherein the electronic device further comprises an i^{th} optical-to-electrical converter, an i^{th} trans-impedance amplifier, an i^{th} analog-to-digital converter, an i^{th} DC adjustment module, and an i^{th} current digital-to-analog converter, wherein i is a positive integer greater than or equal to 2; and
each optical-to-electrical converter is electrically connected to any trans-impedance amplifier and current digital-to-analog converter, and each trans-impedance amplifier is electrically connected to any analog-to-digital converter.

9. The electronic device according to any one of claims 1 to 8, wherein the electronic device further comprises a j^{th} driver and a k^{th} light emitter, wherein both j and k are positive integers greater than or equal to 2; and
each driver is electrically connected to p light emitters, wherein p is a positive integer greater than or equal to 1.

10. The electronic device according to claim 9, wherein in a specific slot, one or more drivers drive one or more light emitters.

11. The electronic device according to any one of claims 1 to 10, wherein the electronic device further comprises a memory, the memory is electrically connected to at least one analog-to-digital converter and is configured to: receive and store a digital signal output by the at least one analog-to-digital converter.

12. A light source dimming method, applied to an electronic device, wherein the electronic device comprises:
one or more processors;
one or more memories;
one or more light emitters;
one or more optical-to-electrical converters; and
one or more DC adjustment modules; and
the method comprises:
in response to a first current adjustment signal, controlling the light emitter to output a first optical signal, and detecting a second optical signal obtained when the first optical signal is transmitted or reflected by a to-be-detected object;
determining, based on the second optical signal, a first photocurrent corresponding to the second optical signal;
obtaining a current transmission ratio between the light emitter and the optical-to-electrical converter based on the first photocurrent and a first drive current of the light emitter;
determining, based on the current transmission ratio, a second drive current of the light emitter and a second photocurrent of the optical-to-electrical converter when the light emitter is driven by using the second drive current to emit light; and
when it is determined that the second photocurrent is within an optimal working range of a target current, adjusting a DC current of the DC adjustment module based on the second photocurrent, for a difference between the second photocurrent and the DC current to be within an optimal working range of the first trans-impedance amplifier in a high-gain mode.

13. The method according to claim 12, wherein the electronic device is further configured to:
determine, based on the current transmission ratio and a target photocurrent, the second drive current that is of the light emitter and that is needed when the target photocurrent is reached; or
determine, based on the current transmission ratio and a pre-stored correspondence between a drive current and a power, the second drive current of the light emitter.

14. The method according to claim 12 or 13, wherein the electronic device is further configured to:
when a difference between the second photocurrent and the target photocurrent is greater than a first threshold, control the DC adjustment module to adjust the DC current, and obtain an adjustment value of the DC current of the first DC adjustment module, wherein there is a correspondence among the adjustment value, the second photocurrent, the target photocurrent, and a convergence threshold of the optimal working range of the target photocurrent, and the first threshold is a DC adjustment trigger threshold.

15. The method according to claim 12 or 13, wherein the electronic device is further configured to:
when a difference between the second photocurrent and the DC current of the DC adjustment module is greater than a second threshold, control the DC adjustment module to adjust the DC current, and obtain an adjustment value of the DC current of the DC adjustment module, wherein there is a correspondence among the adjustment value, the second photocurrent, the DC current of the DC adjustment module, and a convergence threshold of the optimal working range of the target photocurrent.

16. The method according to any one of claims 12 to 15, wherein the electronic device is further configured to:
determine, in an m^{th} slot of an X^{th} cycle, that the second photocurrent exceeds the optimal working range of the target current, wherein both X and m are positive integers greater than or equal to 1; or
determine, in an m^{th} slot of each cycle, that the second photocurrent exceeds the optimal working range of the target photocurrent; or
after the current transmission ratio is measured in a 1^{st} slot, determine, in another slot based on a third drive current generated in the 1^{st} slot, that a photocurrent corresponding to the third drive current exceeds the optimal working range of the target photocurrent; or
after the current transmission ratio is measured in a 1^{st} sub-slot of each slot, determine, based on the current transmission ratio and in a slot of a first cycle to which the 1^{st} sub-slot belongs or a slot of a second cycle to which the 1^{st} sub-slot belongs, that the second photocurrent exceeds the optimal working range of the target current; or
when working is performed in one or more slots of each cycle within a dimming trigger threshold, determine the current transmission ratio, and determine that a current transmission ratio and a second photocurrent that are in another slot exceed the optimal working range of the target current.

17. The method according to any one of claims 12 to 16, wherein the electronic device is further configured to:
determine, in an n^{th} slot of the X^{th} cycle, that the difference between the second photocurrent and the DC current exceeds an optimal working range of a TIA, wherein both X and n are positive integers greater than or equal to 1; or
determine, in an n^{th} slot of each cycle, that the difference between the second photocurrent and the DC current exceeds an optimal working range of a TIA.

18. A computer-readable storage medium, wherein the computer-readable storage medium comprises computer instructions, and when the computer instructions are run on an electronic device, the electronic device is enabled to perform the method according to any one of claims 12 to 17.
